# FASCICULE DE BREVET EUROPEEN

(11) **EP 2 981 619 B1**
(45) Date de publication et mention de la délivrance du brevet: **01.01.2020**
(21) Numéro de dépôt: 14714282.2
(22) Date de dépôt: 01.04.2014
(51) Int. Cl.: C12Q 1/10, C12Q 1/42

(54) **UTILISATION D'AU MOINS UN SUBSTRAT DE PHOSPHATASE CHROMOGÈNE ET/OU FLUOROGÈNE POUR LA DÉTECTION ET/OU LE DÉNOMBREMENT D'ENTÉROBACTÉRIES DANS UN ÉCHANTILLON**
VERWENDUNG MINDESTENS EINES CHROMOGENEN UND/ODER FLUORGENEN PHOSPHATASESUBSTRATS ZUR ERKENNUNG UND/ODER ZÄHLUNG VON ENTEROBAKTERIEN IN EINER PROBE
USE OF AT LEAST ONE CHROMOGENIC AND/OR FLUOROGENIC PHOSPHATASE SUBSTRATE FOR THE DETECTION AND/OR ENUMERATION OF ENTEROBACTERIA IN A SAMPLE

(30) Priorité: 03.04.2013 FR 1353017
(43) Date de publication de la demande: 10.02.2016
(73) Titulaire: bioMérieux, 69280 Marcy-l'Étoile (FR)
(72) Inventeur: CELLIER, Marie, F-38390 Montalieu-Vercieu (FR); BOURGUIGNON, Marie-Pierre, F-01800 Pérouges (FR); HALIMI, Diane, F-01700 Saint Maurice de Beynost (FR); ORENGA, Sylvain, F-01160 Neuville-sur-Ain (FR)
(74) Mandataire: Murgitroyd & Company
(86) Numéro de dépôt international: PCT/EP2014/056544
(87) Numéro de publication internationale: WO 2014/161864

(56) Documents cités:
- EP-A1- 1 790 734
- WO-A1-2004/027086
- WO-A1-2009/026920
- WO-A1-2011/080305
- FR-A1- 2 790 765
- US-A- 4 892 817
- US-A- 5 210 022
- GIUSEPPE SATTA ET AL: "Phosphatase Activity Is a Constant Feature of All Isolates of All Major Species of the Family Enterobacteriaceae", JOURNAL OF CLINICAL MICROBIOLOGY, vol. 26, no. 12, 1 janvier 1988 (1988-01-01), pages 2637-2641, XP055090282,
- David G Bobey ET AL: "Rapid Detection of Yeast Enzymes by Using 4-Methylumbelliferyl Substrates", JOURNAL OF CLINICAL MICROBIOLOGY, vol. 13, no. 2, 1 February 1981 (1981-02-01), pages 393-394, XP055413534,
- MARY BARBER ET AL: "IDENTIFICATION OF STAPHYLOCOCCUS PYOGENES BY THE PHOSPHATASE REACTION", JOURNAL OF PATHOLOGY AND BACTERIOLOGY, vol. 63, no. 1, 1 January 1951 (1951-01-01), pages 65-68, XP055413642, DOI: 10.1002/path.1700630108

## Description

### Domaine technique

La présente invention concerne le domaine de la microbiologie et plus particulièrement celui de la microbiologie industrielle. De manière plus spécifique, la présente invention concerne la détection et/ou le dénombrement des entérobactéries (Eb) dans un échantillon susceptible de (ou suspecté de) les contenir, tel qu'un échantillon alimentaire (produits d'alimentation humaine et animale) ou dans un prélèvement d'environnement de production.

### Etat de la technique

Afin de protéger la santé des consommateurs, les produits commercialisés par l'industrie agro-alimentaire (produits carnés, produits laitiers, produits de la mer, végétaux etc.) sont soumis à de nombreux tests microbiologiques afin de s'assurer de leur innocuité (absence de toxicité). De nombreuses solutions sont couramment mises en œuvre à cet effet, parmi lesquelles :
- La détection et/ou le dénombrement des germes pathogènes à l'aide de milieux de culture, tâches pouvant être automatisées, par exemple à l'aide d'un appareil de type VIDAS®, BacT/ALERT® ou Tempo®,
- Le dénombrement desdits germes pathogènes notamment en utilisant des milieux de culture ad hoc,
- L'identification des micro-organismes, par exemple à l'aide de milieux de culture spécifiques, ou des galeries de type API® / ID 32, ou encore de façon automatisée avec la gamme VITEK®, etc.

Une grande partie des analyses sont réalisées au sein de laboratoires accrédités, contraints d'utiliser des méthodes reconnues conformes aux normes internationales telles que les normes ISO.

Une des normes ISO de premier ordre concernant le contrôle qualité des aliments est la norme ISO 21528-2, intitulée « Microbiologie des aliments -- Méthodes horizontales pour la recherche et le dénombrement des Enterobacteriaceae ». Comme son titre l'indique, la norme précitée s'intéresse à la détection et/ou au dénombrement des entérobactéries. Ces dernières sont en général des hôtes normaux ou pathologiques, suivant les espèces microbiennes, du tube digestif de l'homme et des animaux. Elles semblent plus spécifiquement adaptées à l'homme ou l'animal; certaines sont responsables d'infections humaines parfois sévères (fièvre typhoïde, dysenterie bacillaire, peste). Les entérobactéries sont bien connues de l'homme du métier et ont été caractérisées dans la littérature notamment par leurs caractéristiques bactériologiques (morphologie, comportement en cultures, biochimie, structure antigénique, etc.). D'une manière générale, la définition des entérobactéries peut être considérée comme reposant sur les critères suivants :
- bacilles Gram négatif (Gram-),
- immobiles ou mobiles sur cils péritriches,
- aérobies - anaérobies facultatifs, dégradant le glucose par voie fermentaire (fermentant le glucose), dépourvus d'oxydase et réduisant les nitrates,
- non sporulées.

Beaucoup d'entérobactéries d'origine intestinale peuvent, à des degrés divers, être agressives pour l'homme. On les dit "pathogènes opportunistes". La fréquence de leurs manifestations pathologiques est en augmentation, car souvent due à l'existence, chez ces espèces, de plasmides de résistance aux antibiotiques permettant leur sélection et favorisant à leur avantage les dysmicrobismes. Ce caractère est particulièrement vérifié avec l'espèce *Klebsiella pneumoniae,* hôte des voies respiratoires et qui peut être responsable d'infections.

D'autres entérobactéries sont dites « pathogènes spécifiques » et sont notamment susceptibles de causer des infections digestives (diarrhées, gastroentérites, entérites, dysenterie, adénite mésentérique, fièvres septicémiques, etc.).

A titre d'exemple de « pathogènes spécifiques », l'on peut citer, entre autres, les bactéries appartenant aux genres *Salmonella, Shigella, Yersinia* et *Escherichia coli.*

La publication de SATTA, Giuseppe et al.: «Phosphatase Activity Is a Constant Feature of All Isolates of All Major Species of the Family Enterobacteriaceae», JOURNAL OF MICROBIOLOGY, (1988). Vol. 26, no. 12, pages 2637-2641] divulgue une méthode permettant la détection d'un certain nombre d'entérobactéries en utilisant un substrat de phosphatase, tel que le « phenolphtalein monophosphate » ou le « 6-benzoylnaphtyl phosphate (6-BNP) », et le « methyl green ».

Le brevet US 5,210,022 enseigne notamment l'utilisation d'un substrat de β-galactosidase pour identifier un sous-groupe particulier d'entérobactéries, à savoir les coliformes beta-glucuronidase négative et l'utilisation d'un substrat de beta-glucuronidase afin d'identifier les *Escherichia coli.*

Un milieu de culture connu, à savoir le milieu chromID® Coli (précédemment commercialisé sous la dénomination « Coli ID »; réf. 42 017), vise également à détecter et dénombrer les bactéries coliformes et les *Escherichia coli* (Ec) beta-glucuronidase positive (β-GUR + ; également dénommées « GUR » [Rice *et al.* (1991), von Gravenitz *et al.* (1992)], « beta-Gur » [Looney *et al.* (1990)] ou encore « β-GUS » dans la littérature) dans les produits d'alimentation humaine et animale ainsi que dans les prélèvements d'environnement de production. Il s'agit d'un milieu de culture gélosé en flacon. A toutes fins utiles, il convient de noter que l'activité enzymatique beta-glucuronidase est référencée sous l'intitulé EC 3.2.1.31 au sein de la classification internationale.

Le terme de coliforme est un terme générique regroupant des familles de bactéries différentes et ayant entre autres pour caractéristiques d'être Gram -, oxydase -, et de fermenter le lactose avec production d'acide et de gaz à une température de 30 ou 37°C en présence de sels biliaires (cf. norme ISO 4831 de 1991). Les coliformes font partie de la famille des Enterobactéries mais toutes les entérobactéries ne possèdent pas les caractéristiques susvisées. Sont des coliformes des espèces comme *E.coli* ou certaines espèces des genres *Klebsiella, Enterobacter, Citrobacter.* On les appelle thermotolérants lorsqu'ils possèdent les mêmes propriétés à une température de croissance de 44°C. Peuvent également être considérées comme coliformes des non-Entérobactéries qui possèdent ces propriétés (*Aeromonas* par exemple).

Toutefois, des entérobactéries non-coliformes (telles que les bactéries du genre *Salmonella*) ont un pouvoir pathogène important et, en tant que telles, doivent également faire l'objet de contrôles microbiologiques visant à les détecter et/ou les dénombrer. C'est la raison pour laquelle la norme ISO 21528-2 susmentionnée ne vise pas à détecter/dénombrer uniquement les coliformes mais bien les entérobactéries de manière générale.

Il existe donc un besoin de mettre au point une technique efficace, robuste et facile à mettre en œuvre permettant de détecter et/ou dénombrer les entérobactéries présentes dans un échantillon donné, provenant par exemple d'un lot alimentaire. Préférablement cette technique doit être suffisamment sensible tout en répondant au critère de spécificité requis, à savoir éviter, dans la mesure du possible, la détection et/ou le dénombrement de « faux-positifs », à savoir par exemple la détection et/ou le dénombrement de bactéries Gram négatif non-fermentants.

Un milieu de culture chromogène commercialisé par la société AES Chemunex, le milieu REBECCA®, est regardé comme permettant de dénombrer les *Escherichia coli* (Ec) beta-glucuronidase positive (β-GUR +) et les entérobactéries sur une même boite de Petri. Les colonies formées par les entérobactéries (y compris les éventuelles colonies d'*E*. *coli*) sont colorées en rouge et les colonies de *E. coli* β-GUR + présentent une double coloration, à savoir d'une part ladite coloration rouge, et de l'autre une coloration bleue-verte due au clivage d'un substrat de l'enzyme β-glucuronidase (β-GUR), l'acide 5-bromo-4-chloro-3-indoxyl-beta-D-glucuronique (noté X-GUR, également dénommé acide 5-bromo-4-chloro-3-indolyl-beta-D-glucuronique), par les bactéries *E. coli* β-GUR +. A l'observation, les colonies d'*E. coli* β-GUR + présentent une coloration violacée en leur centre, correspondant au mélange des couleurs rouge et bleu et une coloration bleue sur les bords desdites colonies. Bien que donnant des résultats satisfaisants, ce milieu REBECCA® induit possiblement de « faux-positifs » et peut avoir comme conséquence le blocage et la destruction d'un lot alimentaire en raison d'une suspicion de présence d'entérobactéries. En effet, au-delà d'une quantité ou d'une concentration prédéfinie d'entérobactéries (valeur seuil), les lots d'aliments testés sont bloqués puis détruits. Ces lots ne seront donc pas mis sur le marché et représenteront, par conséquent, une perte financière pour la société qui les commercialise. Or, les résultats positifs obtenus sur milieu REBECCA® (valeur seuil dépassée) peuvent être dus, outre la coloration rouge due aux entérobactéries, à celle attribuable aux bactéries Gram négatif non-fermentantes, généralement moins pathogènes que les premières. En d'autres termes, même si la quantité d'entérobactéries ayant poussé sur le milieu REBECCA® est en valeur absolue inférieure à la valeur seuil, le lot alimentaire sera considéré comme impropre à la consommation du fait des colonies de Gram négatif non-fermentantes détectées, tout comme les entérobactéries, par une coloration rouge sur le milieu REBECCA®. Même si la spécificité de détection du milieu REBECCA® est globalement satisfaisante, il existe tout de même un besoin d'améliorer cette spécificité de détection.

Les normes ayant trait à des milieux pour la détection et/ou le dénombrement (numération) des *Escherichia coli* basées sur la détection d'une activité beta-glucuronidase décrivent des milieux pour la détection et/ou le dénombrement des entérobactéries basés sur l'acidification du glucose, associés à un test de confirmation. En conséquence, pour concevoir un milieu permettant simultanément la détection et/ou le dénombrement des *E. coli* et des entérobactéries, l'homme du métier serait naturellement incité à associer la détection et/ou le dénombrement d'une activité beta-glucuronidase et l'acidification du glucose au sein d'un même milieu de culture. Cependant, cette approche est limitée car les concentrations en glucose nécessaires sont élevées et inhiberaient, selon toute vraisemblance, la détection de l'activité beta-glucuronidase recherchée. Bien évidemment, il est nécessaire que la croissance des entérobactéries susceptibles d'être présentes dans l'échantillon testé ne soit pas inhibée en tout ou partie en raison des substrats et autres réactifs utilisés dans le milieu de culture bactérienne.

### Exposé de l'invention

L'objectif de la présente invention consiste donc à résoudre tout ou partie des problèmes mentionnés supra.

En conséquence, un des objets de la présente invention concerne l'utilisation d'au moins un substrat de phosphatase chromogène et/ou fluorogène pour la détection et/ou le dénombrement d'entérobactéries dans un échantillon susceptible de les contenir (ou suspecté de les contenir), tel qu'un échantillon alimentaire et, de manière simultanée ou séquentielle, d'au moins un substrat de β-glucuronidase chromogène et/ou fluorogène pour identifier, parmi les entérobactéries détectées via ledit substrat de phosphatase, les entérobactéries β-glucuronidase positives, par exemple les *E.coli* β-glucuronidase positives.

En effet, la Demanderesse a découvert, contre toute attente, que les entérobactéries susceptibles d'être présentes dans un échantillon, tel qu'un échantillon alimentaire, avaient en commun une activité enzymatique de type phosphatase. Même si une activité phosphatase a déjà été décrite concernant certaines espèces spécifiques d'entérobactéries, prises individuellement, rien ne laissait penser dans l'art antérieur que cette activité enzymatique de type phosphatase était partagée par l'ensemble des entérobactéries susceptibles d'être retrouvées dans un échantillon et en particulier dans un échantillon alimentaire.

Par « échantillon », l'on entend une petite partie ou petite quantité isolée d'une entité pour l'analyse. Ce peut être un échantillon clinique, humain ou animal, issu d'un prélèvement de liquide biologique, ou un échantillon alimentaire, issu de tout type d'aliment, ou un échantillon de l'environnement de production ou de transformation d'aliments. Cet échantillon peut être ainsi liquide ou solide. L'on peut citer d'une manière non limitative, un échantillon clinique de sang total, de sérum, de plasma, d'urines, de fécès, de prélèvements de nez, de gorges, de peau, de plaies, de liquide céphalo-rachidien, un échantillon alimentaire, d'eau, de boissons tels que le lait, un jus de fruits, de yaourt, de viande, d'œufs, de légumes, de mayonnaise, de fromage, de poisson, un échantillon alimentaire issu d'une alimentation destinée aux animaux, tel que notamment un échantillon issu de farines animales, un échantillon pour contrôle de surface ou d'eau. Ce prélèvement peut être utilisé tel quel ou, préalablement à l'analyse, subir une préparation de type enrichissement, dilution, extraction, concentration, purification, selon des méthodes connues de l'homme du métier.

Par « échantillon alimentaire », l'on entend, au sens de la présente invention, un échantillon issu d'un lot d'aliments donné, sur lequel des contrôles microbiologiques sont susceptibles d'être pratiqués.

Par extrapolation, cet échantillon peut également être dénommé « échantillon biologique », dans la mesure où il est susceptible/suspecté de contenir de la matière biologique (à savoir a minima des entérobactéries).

La détection des entérobactéries permet de déceler, à l'œil nu ou à l'aide d'un appareil optique, l'existence d'une croissance des bactéries cibles (en l'espèce des entérobactéries), à savoir l'apparition de colonies colorées et/ou fluorescentes (selon que l'on utilise un substrat chromogène, fluorogène ou présentant les deux caractéristiques à la fois), lesdites colonies étant colorées et/ou fluorescentes par révélation de l'activité enzymatique de type phosphatase des entérobactéries sur ledit substrat de phosphatase. Généralement, la détection s'opère à l'aide d'un appareil optique pour les substrats fluorogènes, ou à l'œil nu ou à l'aide d'un appareil optique pour les substrats colorés. Avantageusement, la détection peut également permettre une identification des bactéries cibles.

Le dénombrement des entérobactéries consiste, quant à lui, à comptabiliser le nombre de colonies d'entérobactéries ayant poussé sur ce milieu de culture en mettant en œuvre des techniques de microbiologie bien connues de l'homme du métier.

Le recours à un substrat de phosphatase chromogène et/ou fluorogène permet d'obtenir une sensibilité de détection très satisfaisante tout en conférant une très bonne spécificité : la majeure partie des bactéries Gram négatif non-entérobactéries (par exemple les Gram négatif non-fermentants) ne sont pas colorées et/ou fluorescentes.

En outre, un tel substrat de phosphatase inhibe très peu - voire pas du tout - la croissance des entérobactéries sur le milieu de culture, ce qui représente un avantage non-négligeable.

Par substrat chromogène et/ou fluorogène, l'on entend un substrat permettant la détection d'une activité enzymatique ou métabolique des micro-organismes cibles grâce à un signal détectable directement ou indirectement. Pour une détection directe, ce substrat peut être lié à une partie faisant office de marqueur, fluorescent ou coloré (Orenga et al., 2009 ; J. Microbiol. Methods ; 79(2):139-55). Pour une détection indirecte, le milieu réactionnel selon l'invention peut comporter en sus un indicateur de pH, sensible à la variation de pH induite par la consommation du substrat et révélant le métabolisme des micro-organismes cibles. Ledit indicateur de pH peut être un chromophore ou un fluorophore. On citera comme exemples de chromophores le bromocresol pourpre, le bleu de bromothymol, le rouge neutre, le bleu d'aniline, le bleu de bromocresol. Les fluorophores comprennent par exemple la 4-méthylumbelliferone, les dérivés de l'hydroxycoumarine ou les dérivés de la résorufine.

Par « substrat de phosphatase chromogène et/ou fluorogène», il convient de comprendre un substrat enzymatique capable, après réaction avec une enzyme de type phosphatase, de générer un produit coloré et/ou fluorescent. Les réactions enzyme-substrat possibles sont bien connues de l'homme du métier. Il peut s'agir de réactions de type clivage (par exemple par hydrolyse du substrat enzymatique), réduction, etc.

Les substrats de phosphatase chromogènes et/ou fluorogènes utilisés selon la présente invention sont, de préférence, des substrats synthétiques, comprenant deux parties (préférablement constitués en deux parties), une première partie spécifique de l'activité enzymatique à détecter, à savoir une activité enzymatique de type phosphatase (soit un groupement phosphate), et une seconde partie faisant office de marqueur, ci-après appelée partie marqueur chromogène et/ou fluorogène. Ladite partie marqueur est qualifiée de « chromogène et/ou fluorogène », dans la mesure où, lorsqu'elle n'est plus associée à la première partie, à savoir après clivage par l'enzyme phosphatase (par exemple dans le cadre d'une réaction de type hydrolyse) et séparation desdites première et deuxième parties, ladite partie marqueur produit une coloration et/ou une fluorescence. En d'autres termes, ladite partie marqueur chromogène et/ou fluorogène est susceptible, dans certaines conditions, de donner lieu à une réaction colorée et/ou fluorescente.

Les substrats fluorogènes peuvent avoir différentes compositions. Parmi ceux-ci, l'on compte notamment ceux à base de 7-hydroxycoumarine (umbelliferone) ou de 7-aminocoumarine et leurs dérivés substitués en positions 3, 4 (voire 5 et 6) tels que par exemple le 4-methylumbelliferone, qui permettent la libération d'un composé fluorescent de couleur variant du bleu au vert sous lampe à ultraviolets (UV).

D'autres substrats fluorogènes sont à base de résorufine (et ses dérivés). Ils résultent en la libération d'un composé fluorescent rose sous lumière naturelle (λex= 530 nm).

L'on peut également citer les substrats à base de fluorescéine (et ses dérivés) qui, après dégradation, libèrent un composé fluorescent jaune sous lumière naturelle (λex= 485 nm).

En raison de la fluorescence qui ne reste pas localisée au niveau des colonies, les substrats enzymatiques fluorogènes à base des marqueurs cités ci-dessus sont généralement peu adaptés à une utilisation dans des milieux gélosés, et sont plus utilisés en milieu liquide.

Les substrats enzymatiques chromogènes utilisables au sens de la présente invention peuvent être de différentes natures.

Premièrement, il convient de mentionner les substrats à base d'indoxyl et ses dérivés qui, après hydrolyse et en présence d'oxygène, produisent un précipité variant du bleu au rose. Ces substrats à base d'indoxyl et ses dérivés sont particulièrement préférés au sens de la présente invention du fait de leur mise en œuvre relativement aisée et de leur bonne sensibilité dans le cadre de la détection et/ou du dénombrement de bactéries. Leurs applications concernent essentiellement les activités enzymatiques de type osidases, estérases et phosphatases (la phosphatase étant une activité estérase de l'acide phosphorique). Bien adaptés à une utilisation sur support solide ou semi-solide (filtre, gélose, gel d'électrophorèse, etc.), ils le sont moins à une utilisation en milieu liquide (formation d'un précipité).

Certains dérivés d'indoxyl à base d'Aldol® représentent des substrats enzymatiques d'intérêt au sens de la présente invention, dans la mesure où l'apparition d'un précipité coloré ne nécessite aucun ajout (oxygène, sels de métaux, etc.). L'utilisation de tels substrats enzymatiques peut donc s'avérer particulièrement avantageuse dans le cadre d'un ensemencement dans la masse des bactéries. Ces dérivés d'indoxyl à base d'Aldol® sont des dérivés d'indoxyl (1H-indolyl-3-yl) particuliers, à savoir des substrats à base d'indoxyl conjugués sur l'amine cyclique (N-arylés), tels que divulgués dans la demande de brevet PCT publiée sous la référence WO 2010/128120 (au nom de Biosynth AG [CH]). Ces substrats enzymatiques peuvent être obtenus auprès de la société Biosynth AG, et en particulier, peuvent être commandés via le site internet de la société Biosynth AG, à savoir : http://www.biosynth.com.

Deuxièmement, il existe des substrats enzymatiques à base d'hydroxyquinoline, de dihydroxyanthraquinone, de catéchol, de dihydroxyflavone ou d'esculétine et leurs dérivés qui, en présence de sels de fer, produisent un précipité coloré. Là encore, leurs applications concernent essentiellement des activités enzymatiques de type osidases, estérases et phosphatases.

Troisièmement, l'on peut mentionner les substrats enzymatiques à base de nitrophénol et nitroaniline et dérivés, lesquels résultent en la formation d'un composé de couleur jaune. Ils permettent de détecter des activités osidases, estérases et phosphatases dans le cas de substrats à base de nitrophénol et des activités peptidases dans le cas de substrats à base de nitroaniline. Cependant, dans le cas de la détection d'activités peptidases, la nitroaniline libérée est toxique pour les bactéries que l'on souhaite identifier ou caractériser, ce qui peut s'avérer préjudiciable pour des analyses en cours ou ultérieures. D'autre part, ils sont généralement peu adaptés à une utilisation sur support solide et mieux adaptés à une utilisation en milieu liquide. De plus, ils sont peu chromogènes du fait du coefficient d'extinction relativement faible de la couleur (jaune) dont le contraste est peu prononcé dans les milieux biologiques (ce qui influe sur la sensibilité de détection des tests microbiologiques correspondants).

Quatrièmement, il existe des substrats enzymatiques à base de naphthol et naphthylamine et ses dérivés. Dans ce cas, la réaction enzyme-substrat s'effectue en deux temps, le naphthol ou naphthylamine libéré par l'activité enzymatique subit un "azo-coupling" en présence d'un sel de diazonium qui est ajouté au moment de la révélation, conduisant à la formation d'un composé insoluble coloré. Ils permettent de détecter les activités osidases et estérases par l'intermédiaire du naphthol et les activités peptidases par l'intermédiaire de la naphthylamine. La réaction d'"azo-coupling" s'effectue dans un milieu souvent chimiquement agressif, toxique pour les bactéries et rendant l'échantillon inutilisable pour d'autres analyses, de plus les naphtylamines sont cancérigènes.

La présente invention permet, par conséquent, de discriminer sans effort excessif les entérobactéries (colorées et/ou fluorescentes) par rapport aux non-entérobactéries (non-colorées et/ou non-fluorescentes), telles que les Gram négatif non-fermentants. Le fait que la plupart des Gram négatif non-entérobactéries ne soient pas colorées dans le cadre de l'utilisation d'un substrat de phosphatase chromogène et/ou fluorogène dans un échantillon indique une très bonne spécificité du substrat de phosphatase. Ceci représente un avantage significatif par rapport aux différents techniques et milieux développés dans l'art antérieur.

Selon la présente invention, la partie marqueur du substrat de phosphatase chromogène et/ou fluorogène est préférentiellement choisi parmi les substrats à base d'indoxyl (3-Indoxyl, 5-Bromo-3-indoxyl, 5-Iodo-3-indoxyl, 4-Chloro-3-indoxyl, 5-Bromo-4-chloro-3-indoxyl, 5-Bromo-6-chloro-3-indoxyl, 6-Bromo-3-indoxyl, 6-Chloro-3-indoxyl, 6-Fluoro-3 indoxyl, 5-Bromo-4-chloro-N-méthyl-3-indoxyl, N-Méthyl-3-indoxyl, Aldol® ...) ; d'umbelliférone (4-Méthylumbelliférone, Cyclohexenoesculétine, ...) ; d'Alizarine; de p-Naphtolbenzéine; de Nitrophénol (ortho-Nitrophénol, para-Nitrophénol, ...) ; d'Hydroxyquinoline; de Cathécol (Cathécol, Dihydroxyflavone, Hydroxyflavone, ...) ; de Résorufine; de Chlorophénol Red; de Fluorescéine; d'Aminophénol (para-Aminophénol, Dichloro-aminophénol, ...) ; de Naphtol (alpha-Naphtol, 2-Naphtol, Naphtol-ASBI, ...) ; d'Aminocoumarine (7-Amino-4-méthyl-coumarine, ...) ; de Naphtylamide; d'Acridine (Amino-phényl-acridine...) ; d'Amino-phénoxazine (Amino-benzophénoxazinone, Amino-pentyl-resorufine, ...).

De préférence, le substrat de phosphatase chromogène et/ou fluorogène selon la présente invention est sélectionné parmi :
- les substrats à base d'indoxyl ou d'un de ses dérivés, tels que le 5-bromo-4-chloro-3-indoxyl-phosphate (« X-phosphate », abrégé en X-P), le 5-bromo-6-chloro-3-indoxylphosphate (« Magenta phosphate »), le 6-chloro-3-indoxyl-phosphate (« Rose-phosphate », abrégé en Rose-P), le 5-iodo-3-indoxyl-phosphate, le 6-bromo-3-indoxyl-phosphate, le 5,6-dibromo-3-indoxyl-phosphate, le 5-bromo-3-indoxyl-phosphate, le Aldol® 458 phosphate, le Aldol® 470 phosphate, le Aldol® 484 phosphate, le Aldol®495 phosphate, le Aldol®515 phosphate, le Aldol® n phosphate, et
- le 3-hydroxyflavone-phosphate.

Concernant la nomenclature des substrats enzymatiques à base d'indoxyl ou d'un de ses dérivés, il convient de comprendre les racines « indoxyl » et « indolyl » comme étant équivalentes. Ainsi, le X-phosphate peut être indifféremment dénommé «5-bromo-4-chloro-3-indoxyl-phosphate » ou « 5-bromo-4-chloro-3-indolyl-phosphate ».

Tel qu'indiqué supra, le substrat de phosphatase est préférablement un substrat chromogène, avantageusement clivable par l'activité phosphatase des entérobactéries. Ainsi, ce substrat de phosphatase chromogène est, de préférence, un substrat constitué d'une partie cible et d'une partie marqueur. L'hydrolyse du substrat par l'activité phosphatase des entérobactéries induit la séparation (clivage) de la partie cible et de la partie marqueur, ladite partie cible caractérisant l'activité enzymatique phosphatase et ladite partie marqueur étant une molécule permettant de révéler la réaction d'hydrolyse via l'apparition d'une coloration au niveau du site d'hydrolyse (colonies).

Avantageusement, le substrat de phosphatase est un substrat à base d'indoxyl ou d'un de ses dérivés. A titre de substrat de phosphatase à base d'indoxyl, l'on peut notamment citer le 5-bromo-4-chloro-3-indoxyl-phosphate, le 5-bromo-6-chloro-3-indoxyl-phosphate, le 6-chloro-3-indoxyl-phosphate, le 5-iodo-3-indoxyl-phosphate, le 6-bromo-3-indoxyl-phosphate, le 5,6-dibromo-3-indoxyl-phosphate, le 5-bromo-3-indoxyl-phosphate, le Aldol® 458 phosphate, le Aldol® 470 phosphate, le Aldol® 484 phosphate, le Aldol®495 phosphate, le Aldol®515 phosphate, le Aldol® n phosphate. De préférence, l'on utilise le 5-bromo-3-indoxyl phosphate (« Blue-Phosphate », abrégé en Blue-P).

Selon un mode de réalisation préféré, le substrat de phosphatase est un substrat à base d'indoxyl ou d'un de ses dérivés, de préférence choisi parmi le 5-bromo-4-chloro-3-indoxyl-phosphate, le 5-bromo-6-chloro-3-indoxyl-phosphate, le 6-chloro-3-indoxyl-phosphate, le 5-iodo-3-indoxyl-phosphate, le 6-bromo-3-indoxyl-phosphate, le 5,6-dibromo-3-indoxyl-phosphate, le 5-bromo-3-indoxyl-phosphate, ledit substrat de phosphatase étant utilisé en combinaison avec un agent favorisant la polymérisation oxydative du dérivé indoxyl, tel qu'un complexe métallique de type citrate de fer ammoniacal. Cette réaction de polymérisation du dérivé indoxyl conduit à la formation d'un composé coloré insoluble.

L'association d'un substrat de phosphatase à base d'indoxyl ou d'un de ses dérivés et d'un agent favorisant la polymérisation oxydative du dérivé indoxyl, tel qu'un complexe métallique de type citrate de fer ammoniacal est, en règle générale, recommandée (voire nécessaire) pour tous les substrats de phosphatase à base d'indoxyl ou d'un de ses dérivés, à l'exception des dérivés d'indoxyl à base d'Aldol®, tels que divulgués dans la demande de brevet PCT publiée sous la référence WO 2010/128120, pour lesquels la génération de la couleur et/ou de la fluorescence est dite « spontanée », à savoir ne nécessite ni oxydation, ni ajout dudit agent favorisant la polymérisation oxydative du dérivé indoxyl, tel qu'un complexe métallique de type citrate de fer ammoniacal. Ces substrats à base d'Aldol®, tels que le Aldol® 458 phosphate, le Aldol® 470 phosphate, le Aldol® 484 phosphate, le Aldol®495 phosphate, le Aldol®515 phosphate, le Aldol® n phosphate, représentent donc des substrats de phosphatase particulièrement préférés au sens de la présente invention, notamment dans des conditions de faible pression en dioxygène (par exemple en microaérophilie), pouvant aller jusqu'à l'anaérobiose. Comme indiqué supra, ceux-ci peuvent être obtenus auprès de la société Biosynth AG, et en particulier peuvent être commandés via le site internet de la société Biosynth AG, à savoir : http://www.biosynth.com. Les références catalogues (consultables notamment sur le site internet susvisé) des différents Aldol® mentionnés supra sont listées au sein du tableau A, présenté ci-après :

**Tableau A**

| Dénomination | Référence catalogue BIOSYNTH® |
|---|---|
| Aldol® 470 phosphate, sel de disodium | A-4678_P00 |
| Aldol® 458 phosphate, sel de disodium | A-4694_P00 |
| Aldol® 495 phosphate, sel de disodium | A-4704_P00 |
| Aldol® 515 phosphate, sel de disodium | A-4721_P00 |

A titre illustratif, les substrats Aldol® 470 phosphate, Aldol® 495 phosphate et Aldol® 515 phosphate sont définis par la formule générale (I) représentée ci-après : 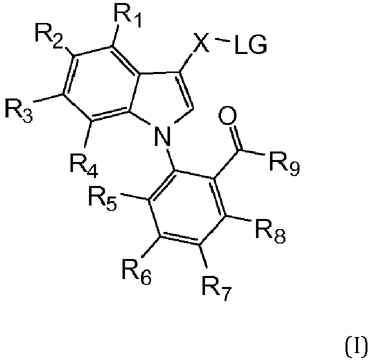 dans laquelle les groupements R1, R2, R3, R4, R5, R6, R7, R8, R9, X et LG sont tels que définis dans le tableau B infra :

**Tableau B**

| Dénomination | R1 | R2 | R3 | R4 | R5 | R6 | R7 | R8 | R9 | X | LG |
|---|---|---|---|---|---|---|---|---|---|---|---|
| Aldol® 470 phosphate | H | H | H | H | H | H | H | H | H | R12 | phosphate |
| Aldol® 495 phosphate | H | H | Cl | H | H | H | H | H | H | R13 | phosphate |
| Aldol® 515 phosphate | H | H | H | H | H | H | H | H | H | R14 | phosphate |

avec

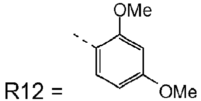

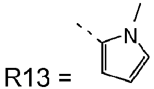

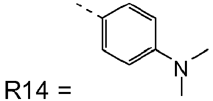

A titre « d'agents favorisant la polymérisation oxydative du dérivé indoxyl du substrat de phosphatase », l'on peut citer, en sus du citrate de fer ammoniacal et à titre d'exemples, le permanganate de potassium et le ferrycyanide de potassium.

L'agent favorisant la polymérisation oxydative du dérivé indoxyl (par exemple un complexe métallique de type citrate de fer ammoniacal) est utilisé, de préférence, à une concentration comprise entre environ 0,05 et environ 0,9 mg/ml, de préférence de l'ordre de 0,3 mg/ml.

L'agent favorisant la polymérisation oxydative du dérivé indoxyl (de préférence un complexe métallique oxydant) permet notamment d'intensifier les couleurs obtenues au sein des colonies en catalysant la polymérisation oxydative du dérivé indoxyl, conduisant ainsi à la formation d'un composé coloré insoluble.

La concentration à laquelle le substrat de phosphatase chromogène et/ou fluorogène doit être utilisé dans le milieu de culture comprenant l'échantillon est aisément déterminable par l'homme du métier sur la base de ses connaissances générales et, le cas échéant, de tests de routines. Cette concentration doit être suffisante pour atteindre le niveau de sensibilité de détection requis mais ne doit pas être trop importante afin de ne pas risquer d'inhiber la croissance des entérobactéries. A titre d'exemple, la concentration de ce substrat de phosphatase dans le milieu de culture comprenant l'échantillon à tester est comprise entre environ 0,05 et environ 0,3 g/L, de préférence entre environ 0,15 et 0,25 g/L, avantageusement de l'ordre de 0,175 g/L.

Selon un mode de réalisation particulièrement préféré, le substrat de phosphatase est contenu dans une composition comprenant au moins un, de préférence deux, et avantageusement trois des constituants suivants :
- un milieu de culture bactérien adapté aux bactéries à détecter (entérobactéries),
- un système sélectif anti-Gram positif,
- un agent antifongique.

Avantageusement, ladite composition comprend un milieu de culture bactérien adapté aux bactéries à détecter (entérobactéries) et au moins un, et de préférence deux, des deux constituants suivants :
- un système sélectif anti-Gram positif,
- un agent antifongique.

Selon un mode de réalisation particulièrement préféré, cette composition est un milieu réactionnel chromogène et/ou fluorogène. Ce dernier permet la détection et/ou le dénombrement des entérobactéries dans un échantillon susceptible (ou suspecté) de contenir des entérobactéries.

Par « milieu réactionnel », l'on entend un milieu comprenant tous les éléments nécessaires à l'expression d'un métabolisme et/ou à la croissance de micro-organismes. Le milieu réactionnel peut être solide, semi-solide ou liquide. Par milieu solide, l'on entend par exemple un milieu gélifié. L'agar est l'agent gélifiant traditionnel en microbiologie pour la culture des micro-organismes, mais il est possible d'utiliser de la gélatine, de l'agarose ou d'autres gélifiants naturels ou artificiels. Un certain nombre de préparations sont disponibles dans le commerce, comme par exemple l'agar Columbia, la gélose Trypcase-soja, la gélose Mac Conkey, la gélose Mueller Hinton ou plus généralement celles décrites dans le Handbook of Microbiological Media (CRC Press). Ledit milieu réactionnel peut comprendre un ou plusieurs éléments en combinaison, tels que des acides aminés, des peptones, des hydrates de carbone, des nucléotides, des minéraux, des vitamines, etc. Le milieu peut comprendre également un colorant. A titre indicatif, on peut citer comme colorant le bleu d'Evans, du rouge neutre, du sang de mouton, du sang de cheval, un opacifiant tel que l'oxyde de Titane, de la nitroaniline, du vert malachite, du vert brillant, un ou plusieurs indicateurs métaboliques, un ou plusieurs régulateurs métaboliques etc.

Au sens de la présente invention, le milieu réactionnel consiste à la fois en un milieu de culture et de révélation.

Concernant le milieu de culture bactérien adapté aux bactéries à détecter, celui-ci sera choisi sans difficulté par l'homme du métier comme un milieu de culture favorisant la croissance des entérobactéries.

Selon un mode de réalisation préféré, l'on effectue un ensemencement en masse, à savoir l'on disperse 1 mL de l'échantillon à tester au fond d'une boîte de Pétri puis l'on coule le milieu de culture (par exemple milieu gélosé) par-dessus. Les entérobactéries se développent alors dans la masse du milieu gélosé et donnent lieu à des colonies. Dans le cadre de cet ensemencement en masse, les entérobactéries sont cultivées dans des conditions de microaérophilie.

L'emploi d'au moins un système sélectif anti-Gram positif (anti-Gram+) et/ou d'au moins un agent antifongique (préférablement des deux) est recommandé au sens de la présente invention car il permet d'améliorer la sélectivité.

Les systèmes sélectifs anti-Gram positif (anti-Gram+) et/ou antifongiques sont bien connus de l'homme du métier. A titre d'exemple, dans le système sélectif anti-Gram positif utilisé aux fins de la présente invention, l'on utilise un ou plusieurs agent(s) sélectif(s) sélectionné(s) dans le groupe constitué par : les sels biliaires (à titre d'exemple le desoxycholate de sodium, le glycocholate de sodium, ou encore un mélange de plusieurs sels biliaires), le cristal violet, le Tergitol® 4 ou Niaproof® 4. De préférence, l'on utilise du desoxycholate et du cristal violet à des concentrations connues de l'homme du métier pour obtenir l'effet escompté, à savoir l'élimination des bactéries Gram positives.

Par « agent sélectif », l'on entend tout composé susceptible d'empêcher ou de ralentir la croissance d'un micro-organisme autre que le micro-organisme cible. Sans être limitatif, une concentration comprise entre 0,01 mg/l et 5 g/l en agent(s) sélectif(s) est particulièrement adaptée à la présente invention.

Par antifongique (« ou agent antifongique »), l'on entend tout composé susceptible d'empêcher ou de ralentir la croissance d'une levure ou d'une moisissure. A titre indicatif, l'on peut citer notamment l'amphotéricine B, le fluconazole, l'itraconazole, le voriconazole, la cycloheximide. De préférence, l'on utilise au moins un agent antifongique à des concentrations connues de l'homme du métier pour obtenir l'effet susvisé.

Par « antibiotique », l'on entend tout composé susceptible d'empêcher ou de ralentir la croissance d'une bactérie. Les antibiotiques appartiennent notamment aux groupes des beta-lactamines, des glycopeptides, des aminosides, des polypeptides, des sulfamides, des quinolones. A titre indicatif, l'on peut citer notamment les antibiotiques cefotaxime, cefsulodine, ceftazidime, cefoxitine, ceftriaxone, cefpodoxime, aztréonam, vancomycine, gentamicine, Triméthoprime, tobramycine, moxalactam, fosfomycine, D-cylcosérine, Polymixine, Colistine, les quinolones telles que l'acide nalidixique.

Les entérobactéries susceptibles d'être présentes dans un échantillon - et plus particulièrement dans un échantillon d'origine alimentaire - sont connues de l'homme du métier. A titre d'exemple, l'on peut citer les entérobactéries comprises dans le groupe constitué par: *E. coli, Salmonella, Klebsiella, Citrobacter, Yersinia* etc. Tel qu'indiqué précédemment, il a été découvert par la Demanderesse, contre toute attente, que ces entérobactéries avaient en commun une activité phosphatase.

La composition selon l'invention (milieu chromogène et/ou fluorogène selon un mode de réalisation préféré) peut, bien évidemment, comprendre d'autres constituants afin d'optimiser la détection et/ou le dénombrement des entérobactéries dans un échantillon. L'homme du métier sera, à cet égard, tout à fait capable de sélectionner ces constituants additionnels. A titre d'exemple, ces derniers peuvent être du sulfate de magnésium à une concentration comprise entre 5 mM et 100 mM, de préférence 20 mM, et/ou au moins un antibiotique.

L'invention a pour objet l'utilisation d'au moins un substrat de phosphatase chromogène et/ou fluorogène pour la détection et/ou le dénombrement d'entérobactéries dans un échantillon susceptible de les contenir, tel qu'un échantillon alimentaire et, de manière simultanée ou séquentielle, d'au moins un substrat de β-glucuronidase chromogène et/ou fluorogène pour identifier, parmi les entérobactéries détectées via ledit substrat de phosphatase, les entérobactéries β-glucuronidase positives, par exemple les *E.coli* β-glucuronidase positives.

En effet, outre la présence et le nombre de colonies d'entérobactéries, un autre facteur essentiel à prendre en compte dans le cadre du contrôle qualité d'un aliment à destination humaine ou vétérinaire est le nombre d'entérobactéries β-glucuronidase positives. En effet, ces entérobactéries β-glucuronidase positives sont connues pour posséder un important pouvoir pathogène. Ceci est particulièrement vrai en ce qui concerne les *Escherichia coli* β-glucuronidase positives. Leur détection est d'ailleurs encadrée par la norme ISO 16649-2, intitulée « Microbiologie des aliments -- Méthode horizontale pour le dénombrement des Escherichia coli bêta-glucuronidase positive ».

L'invention répond donc à une double exigence : détecter et/ou dénombrer les entérobactéries totales dans un échantillon d'une part, et identifier et/ou dénombrer, parmi ces entérobactéries totales, les entérobactéries β-glucuronidase positives, potentiellement pathogènes, voire hautement pathogènes, d'autre part.

Tel qu'indiqué précédemment, les colonies d'entérobactéries sont détectées par l'apparition de réactions d'une première couleur et/ou fluorescence produites par l'activité phosphatase des entérobactéries sur le substrat de phosphatase. Les entérobactéries β-glucuronidase positives (par exemple les *E.coli* β-glucuronidase positives) exhibent une « tierce coloration et/ou fluorescence » (couleur et/ou fluorescence composite) due au mélange de couleurs et/ou de fluorescences entre la première couleur et/ou fluorescence et la deuxième couleur et/ou fluorescence produite par l'activité β-glucuronidase des entérobactéries β-glucuronidase positives.

Ceci permet de visualiser (de visu ou via un dispositif électronique tel qu'une caméra ou un appareil photographique), dans un échantillon mis en culture, à la fois les entérobactéries, d'une manière générale, mais également les entérobactéries β-glucuronidase positives (en particulier les E. coli β-glucuronidase positives), et ce avec une spécificité accrue par rapport aux milieux utilisés dans l'art antérieur (tel que le milieu REBECCA®). Par spécificité, l'on entend la capacité à donner un résultat négatif lorsque la souche bactérienne cible n'est pas présente. En d'autres termes, selon la présente invention, une identification plus spécifique correspond à une réduction du nombre de faux positifs liés aux souches n'exprimant pas les activités enzymatiques recherchées, sans prétendre inhiber l'ensemble de ces souches.

L'utilisation d'au moins un substrat de phosphatase et d'au moins un substrat de β-glucuronidase permet la détection et/ou le dénombrement des entérobactéries (présentant une première couleur et/ou fluorescence) et, parmi ces dernières, des entérobactéries β-glucuronidase positives (présentant une tierce coloration et/ou fluorescence, mélange de ladite première coloration et/ou fluorescence et d'une deuxième coloration et/ou fluorescence) à partir d'un échantillon (par exemple un échantillon alimentaire). De surcroît, la détection et/ou le dénombrement d'une activité phosphatase et d'une activité beta-glucuronidase peut être réalisée simultanément dans un même milieu, sans inhibition de l'une par le substrat de l'autre, c'est-à-dire sans inhibition de l'activité beta-glucuronidase par le substrat de phosphatase, ni de l'activité phosphatase par le substrat de beta-glucuronidase.

En outre, ni le substrat de phosphatase, ni le substrat de β-glucuronidase n'inhibent la croissance des bactéries lors de l'étape de mise en culture de l'échantillon (par exemple prélevé à partir d'un échantillon d'aliments donné). Ceci permet d'effectuer des contrôles microbiologiques fiables et robustes.

La définition du substrat de phosphatase, au sens de la présente invention, s'applique *mutatis mutandis* à celle du substrat de beta-glucuronidase en ce qui concerne le choix de sa partie marqueur. Ainsi, par « substrat de β-glucuronidase chromogène et/ou fluorogène», il convient de comprendre un substrat enzymatique capable, après réaction avec une enzyme de type β-glucuronidase, de donner lieu à une réaction colorée et/ou fluorescente. Les réactions enzyme-substrat possibles sont bien connues de l'homme du métier. Il peut s'agir de réactions de type clivage (par exemple par hydrolyse du substrat enzymatique), réduction, etc.

Les substrats de β-glucuronidase chromogènes et/ou fluorogènes utilisés selon la présente invention sont, de préférence, des substrats synthétiques, comprenant deux parties (préférablement constitués en deux parties), une première partie spécifique de l'activité enzymatique à détecter, à savoir une activité enzymatique de type β-glucuronidase, et une seconde partie faisant office de marqueur, ci-après appelée partie marqueur chromogène et/ou fluorogène. Ladite partie marqueur est qualifiée de « chromogène et/ou fluorogène », dans la mesure où, lorsqu'elle n'est plus associée à la première partie, à savoir après clivage par l'enzyme β-glucuronidase (par exemple dans le cadre d'une réaction de type hydrolyse) et séparation desdites première et deuxième parties, ladite partie marqueur produit une coloration et/ou une fluorescence. En d'autres termes, ladite partie marqueur chromogène et/ou fluorogène est susceptible, dans certaines conditions, de donner lieu à une réaction colorée et/ou fluorescente.

Selon la présente invention, la partie marqueur du substrat de β-glucuronidase chromogène et/ou fluorogène est préférentiellement choisie parmi les substrats à base d'indoxyl (3-Indoxyl, 5-Bromo-3-indoxyl, 5-Iodo-3-indoxyl, 4-Chloro-3-indoxyl, 5-Bromo-4-chloro-3-indoxyl, 5-Bromo-6-chloro-3-indoxyl, 6-Bromo-3-indoxyl, 6-Chloro-3-indoxyl, 6-Fluoro-3 indoxyl, 5-Bromo-4-chloro-N-méthyl-3-indoxyl, N-Méthyl-3-indoxyl, Aldo®...); d'umbelliférone (4-Méthylumbelliférone, Cyclohexenoesculétine, ...) ; d'Alizarine; de p-Naphtolbenzéine; de Nitrophénol (ortho-Nitrophénol, para-Nitrophénol, ...) ; d'Hydroxyquinoline; de Cathécol (Cathécol, Dihydroxyflavone, Hydroxyflavone, ...) ; de Résorufine; de Chlorophénol Red; de Fluorescéine; d'Aminophénol (para-Aminophénol, Dichloro-aminophénol, ...) ; de Naphtol (alpha-Naphtol, 2-Naphtol, Naphtol-ASBI, ...) ; d'Aminocoumarine (7-Amino-4-méthyl-coumarine, ...) ; de Naphtylamide; d'Acridine (Amino-phényl-acridine...) ; d'Amino-phénoxazine (Amino-benzophénoxazinone, Amino-pentyl-resorufine, ...).

De préférence, les substrats enzymatiques de β-glucuronidase chromogènes et/ou fluorogènes utilisés selon la présente invention sont sélectionnés parmi :
- le 4 méthylumbelliféryl-beta-glucuronide,
- les substrats à base d'indoxyl ou d'un de ses dérivés tels que le 5 bromo-4 chloro-3 indoxyl-beta-glucuronide, le 5 bromo-6 chloro-3 indoxyl-beta-glucuronide, le 6 chloro-3 indoxyl-beta-glucuronide, de préférence le 6-chloro-3-indoxyl- β - glucuronide,
- l'alizarine-beta-glucuronide et le cyclohexenoesculetine-beta-glucuronide ou leurs sels,
de préférence ledit au moins un substrat de beta-glucuronidase étant au moins un substrat à base d'indoxyl ou d'un de ses dérivés.

Avantageusement le substrat de beta-glucuronidase est le 6-chloro-3-indoxyl- β - glucuronide (« Rose β-Glucuronide »).

Tout comme le substrat de phosphatase, le substrat de beta-glucuronidase est préférablement un substrat chromogène, avantageusement clivable par l'activité β-glucuronidase des entérobactéries. Ainsi, ce substrat de β-glucuronidase est, de préférence, un substrat constitué d'une partie cible et d'une partie marqueur. L'hydrolyse du substrat par l'activité β-glucuronidase des entérobactéries β-glucuronidase positives induit la séparation (clivage) de la partie cible et de la partie marqueur, ladite partie cible caractérisant l'activité enzymatique β-glucuronidase et ladite partie marqueur étant une molécule permettant de révéler la réaction d'hydrolyse via une réaction colorée.

Tout comme le 5-bromo-3-indoxyl phosphate (« Blue-Phosphate »), le 6-chloro-3-indoxyl-beta-glucuronide (« Rose-beta-Glucuronide ») permet d'obtenir une sensibilité de détection très satisfaisante et, par conséquent, de détecter aisément les entérobactéries beta-glucuronidase positives.

Lorsque l'on utilise le couple préféré de substrats enzymatiques 5-bromo-3-indoxyl phosphate et 6-chloro-3-indoxyl-beta-glucuronide, les entérobactéries autres que les β-glucuronidase positives sont colorées en bleu (clivage du « Blue-Phosphate »), la majeure partie des Gram- non-entérobactéries (par exemple Gram- non-fermentants) ne sont pas colorés (absence d'activité phosphatase et β-glucuronidase) et les entérobactéries β-glucuronidase positives (par exemple les E. coli β-glucuronidase positives) apparaissent « violacées » (couleur variant du rose foncé au violet). La coloration violacée est due au mélange des couleurs bleues et roses (clivage à la fois du « Blue-Phosphate » et du « Rose-beta-Glucuronide » par les entérobactéries β-glucuronidase positives possédant les deux activités enzymatiques).

Selon un mode de réalisation particulièrement préféré, le substrat de phosphatase et/ou le substrat de β-glucuronidase est/sont un/des substrat(s) à base d'indoxyl ou un de ses dérivés utilisé(s) en combinaison avec un agent favorisant la polymérisation oxydative du dérivé indoxyl tel qu'un complexe métallique de type citrate de fer ammoniacal.

A titre de substrat de β-glucuronidase à base d'indoxyl, l'on peut citer le 5 Bromo-4 chloro-3 indoxyl-beta-glucuronide, le 5 Bromo-6 chloro-3 indoxyl-beta-glucuronide, le 6 Chloro-3 indoxyl-beta-glucuronide (« Rose β-Glucuronide »), ce dernier composé étant le substrat de beta-glucuronidase préféré, tel qu'indiqué précédemment.

L'agent favorisant la polymérisation oxydative du dérivé indoxyl est tel que défini précédemment et est utilisé aux concentrations susvisées.

La concentration à laquelle le substrat de β-glucuronidase chromogène et/ou fluorogène doit être utilisé dans le milieu de culture comprenant l'échantillon est aisément déterminable par l'homme du métier sur la base de ses connaissances générales et, le cas échéant, de tests de routine. Cette concentration doit être suffisante pour atteindre le niveau de sensibilité de détection requis mais ne doit pas être trop importante afin de ne pas risquer d'inhiber la croissance des entérobactéries. A titre d'exemple, la concentration de ce substrat de β-glucuronidase dans le milieu de culture comprenant l'échantillon à tester est comprise entre environ 0,1 et environ 0,3 g/L, de préférence entre environ 0,15 et 0,25 g/L, avantageusement de l'ordre de 0,175 g/L.

De préférence, ledit substrat de phosphatase et ledit substrat de β-glucuronidase sont contenus dans une composition comprenant au moins un, de préférence deux, et avantageusement trois des constituants suivants :
- un milieu de culture bactérien adapté aux bactéries à détecter (entérobactéries),
- un système sélectif anti-Gram positif,
- un agent antifongique.

Chacun des trois constituants susvisés est tel que défini précédemment.

Avantageusement, ladite composition comprend un milieu de culture bactérien adapté aux bactéries à détecter (entérobactéries) et au moins un, et de préférence deux, des deux constituants suivants :
- un système sélectif anti-Gram positif,
- un agent antifongique.

Est également décrite la composition *perse,* telle que définie précédemment, à savoir comprenant au moins un substrat de phosphatase chromogène et/ou fluorogène et au moins un substrat de β-glucuronidase chromogène et/ou fluorogène.

Selon un mode de réalisation particulièrement préféré, cette composition est un milieu réactionnel chromogène et/ou fluorogène, consistant à la fois en un milieu de culture et de révélation.

Comme expliqué au fil de la présente demande, cette composition permet de détecter la présence des entérobactéries dans un échantillon via leur activité phosphatase et le substrat de β-glucuronidase permet d'identifier, parmi lesdites entérobactéries ainsi détectées, les entérobactéries β-glucuronidase positives via leur activité β-glucuronidase, par exemple les *E. coli* β-glucuronidase positives.

Avantageusement, le substrat de phosphatase et/ou le substrat de β-glucuronidase est/sont un/des substrat(s) à base d'indoxyl ou un de ses dérivés, ladite composition comprenant un agent favorisant la polymérisation oxydative du dérivé indoxyl dudit substrat de phosphatase et/ou dudit substrat de β-glucuronidase, tel qu'un complexe métallique de type citrate de fer ammoniacal.

Un autre objet de la présente invention concerne l'utilisation de ladite composition pour détecter la présence des entérobactéries dans un échantillon via leur activité phosphatase identifier, parmi les entérobactéries ainsi détectées, les entérobactéries β-glucuronidase positives via leur activité β-glucuronidase, par exemple les *E. coli* β-glucuronidase positives.

Est également décrit un procédé de détection et/ou le dénombrement des entérobactéries dans un échantillon susceptible de contenir lesdites entérobactéries, tel qu'un échantillon alimentaire, ledit procédé comprenant les étapes suivantes :
a) mettre en présence ledit échantillon avec au moins un substrat de phosphatase chromogène et/ou fluorogène,
b) incuber à une température et durant un laps de temps suffisants pour permettre la croissance des entérobactéries susceptibles d'être présentes dans l'échantillon,
c) détecter et/ou dénombrer les colonies d'entérobactéries par l'apparition de réactions colorées et/ou fluorogènes, lesdites réactions colorées et/ou fluorogènes étant produites par l'activité phosphatase des entérobactéries sur ledit au moins un substrat de phosphatase.

Le ou les substrats de phosphatase utilisé(s) est/sont tel(s) que défini(s) précédemment.

L'homme du métier saura, sur la base de ses connaissances générales et, au besoin, de tests de routine, déterminer aisément les paramètres température et temps optimaux concernant la phase d'incubation b). De préférence, cette phase d'incubation consiste à porter et à maintenir l'échantillon (et éventuellement un milieu de culture approprié), entre 1 et 48 heures, préférentiellement entre 4 et 24 heures, plus préférentiellement entre 16 et 24 heures, à une température appropriée (déterminable par l'homme du métier), généralement comprise entre 20 et 50°C, préférentiellement entre 30 et 40°C.

Selon un mode de réalisation préféré, l'étape a) s'effectue en mettant en contact ledit échantillon avec une composition comprenant ledit substrat de phosphatase et au moins un milieu de culture bactérien.

De préférence, ladite composition comprend au moins un système sélectif anti-Gram positif et/ou au moins un antifongique, par exemple ladite composition comprenant au moins un système sélectif anti-Gram positif et un au moins un antifongique.

Selon l'invention, à l'étape a), l'échantillon est mis en présence avec une composition comprenant au moins un substrat de phosphatase chromogène et/ou fluorogène et au moins un substrat de β-glucuronidase chromogène et/ou fluorogène, et l'on détecte, à l'étape c), l'apparition de réaction(s) d'une première couleur et/ou fluorescence produites par l'activité phosphatase des entérobactéries sur ledit substrat de phosphatase et, de manière simultanée ou séquentielle (de préférence simultanée), l'apparition de réactions d'une deuxième couleur et/ou fluorescence produites par l'activité β-glucuronidase des entérobactéries β-glucuronidase positives, par exemple des *E.coli* β-glucuronidase positives. L'étape d'incubation b), quant à elle, est effectuée à une température et pendant un temps connus de - ou aisément déterminables par - l'homme du métier, aptes à permettre une réaction détectable, à l'étape c),

Le ou les substrats de β-glucuronidase utilisé(s) est/sont tel(s) que défini(s) précédemment.

De préférence, l'étape a) du procédé selon l'invention s'effectue en mettant en culture ledit échantillon avec une composition comprenant ledit substrat de phosphatase, ledit substrat de β-glucuronidase et au moins un milieu de culture bactérien.

L'invention a également pour objet un procédé de détection et/ou de dénombrement d'entérobactéries dans un échantillon susceptible de les contenir, ledit procédé comprenant les étapes suivantes :
a) Prélever ledit échantillon,
b) Mettre en contact l'échantillon prélevé à l'étape a) avec une composition comprenant au moins un substrat de phosphatase chromogène et/ou fluorogène et au moins un substrat de β-glucuronidase chromogène et/ou fluorogène,
c) Eventuellement incuber l'ensemble à une température et durant un laps de temps adaptés pour permettre la croissance desdites entérobactéries,
d) Détecter et/ou dénombrer :
   - les entérobactéries, de préférence leur(s) colonie(s), présentant une première coloration et/ou fluorescence produite(s) par l'activité phosphatase desdites entérobactéries sur ledit substrat de phosphatase et, de manière simultanée ou séquentielle, de préférence de manière simultanée,
   - les entérobactéries β-glucuronidase positives, par exemple les *E.coli* β-glucuronidase positives, de préférence leur(s) colonie(s), présentant une coloration et/ou fluorescence composite produite(s) par le mélange de ladite première coloration et/ou fluorescence et d'une deuxième coloration et/ou fluorescence produite(s) par l'activité β-glucuronidase desdites entérobactéries β-glucuronidase positives.

Avantageusement ce procédé de détection et/ou de dénombrement comprend l'étape d'incubation c).

### Description détaillée de l'invention

### Exemple 1 : Spécificité des substrats de phosphatase pour les entérobactéries

### 1.1. Milieux et micro-organismes

38 souches bactériennes, parmi lesquelles 31 entérobactéries et 7 non-entérobactéries, ont été testées sur des milieux contenant différents substrats de phosphatase. La lecture des boites est ensuite effectuée à 24h.

La composition des milieux est présentée dans le Tableau 1 ci-dessous :

**Tableau 1**

| *Base commune à tous les milieux (base milieu chromID® Coli ref 42017* - *mentionné en préambule :* | |
|---|---|
| **Composés** | **Conc. en g/L** |
| **Peptone de gélatine porcine** | **5,5** |
| **Extrait de levure** | **8** |
| **agar européen** | **12,5** |
| **Chlorure de sodium** | **5** |
| **Désoxycholate de sodium** | **0,7** |
| **Cristal violet** | **0,001** |
| **Niaproof 4** | **0,55 ml/l** |

Ensuite, l'on ajoute des substrats de phosphatase comme suit :
- Milieu 1 : ajout du X-phosphate (X-P) à 60 mg/l
- Milieu 2 : ajout du Rose-phosphate (Rose-P) à 300 mg/L
- Milieu 3 : ajout du Magenta-phosphate (Magenta-P) à 300 mg/L
- Milieu 4 : ajout du Purple-phosphate (Purple-P) à 200 mg/L

### 1.2. Essai

Les milieux sont répartis en boites de Pétri.

L'ensemencement est effectué à partir de pré-cultures de 24h à 30°C ou 37°C, selon les espèces, sur milieu Trypcase soja.

Une suspension en eau physiologique à 0,5 McF est réalisée puis les souches sont ensemencées à l'oese calibrée de 10µl.

Des lectures sont effectuées après 24h et 48h d'incubation à 37°C.

### 1.3. Résultats

Ainsi, après 24 et 48h d'incubation, l'on obtient :
- Une coloration verte des colonies sur le milieu 1,
- Une coloration rose des colonies sur le milieu 2,
- Une coloration violette des colonies sur les milieux 3 et 4.

Ont été considérées comme positives, les colonies dont les intensités de coloration sont supérieures ou égales à 1, en appliquant l'échelle de lecture suivantes.

### Echelle de lecture de l'activité enzymatique/ coloration

- 0= pas d'activité
- 0,5 = coloration très pâle
- 1= coloration nette de faible intensité
- 2 = coloration franche intensité moyenne,
- 3 = présence d'une coloration intense,
- 4 = présence d'une coloration très intense.

Les résultats sont présentés dans le Tableau 2 ci-dessous :

**Tableau 2**

| | | | X-P | Rose-P | Magenta-P | Purple-P |
|---|---|---|---|---|---|---|
| Entérobactéries | 24h | croissance | 30/31 | 30/31 | 30/31 | 26/31 |
| | | coloration >= 1 | **90%** | **77%** | **90%** | **83%** |
| | 48h | croissance | 31/31 | 31/31 | 31/31 | 31/31 |
| | | coloration | **97%** | **94%** | **100%** | **100%** |
| % Gram négatif non fermentant positifs | 24h | croissance | 7/7 | 5/7 | 5/7 | 5/7 |
| | | coloration >= 1 | **0%** | **0%** | **0%** | **0%** |
| | 48h | croissance | 7/7 | 7/7 | 7/7 | 7/7 |
| | | coloration >= 1 | **0%** | **0%** | **0%** | **0%** |

### 1.4. Interprétation

En fonction du substrat utilisé, on obtient au minimum, à 24h, une coloration pour 77% des souches d'entérobactéries et, à 48h, une coloration pour, au minimum, 94% de celles-ci. Les substrats de X-phosphate et de Magenta-phosphate semblent être les plus sensibles à 24h. A 48h, les résultats obtenus sont sensiblement identiques pour le X-phosphate, le Magenta-phosphate et le Purple-phosphate. Le Rose-phosphate est le substrat le moins sensible à 24h et 48h.

Sur les 7 souches à Gram négatif non-fermentantes, aucune n'a donné de coloration supérieure ou égale à 1, quel que soit le substrat utilisé.

### 1.5. Conclusion

La très bonne sensibilité et la très bonne spécificité des substrats de phosphatase vis-à-vis des entérobactéries en font, de manière surprenante, des marqueurs de choix pour la détection et/ou le dénombrement de ces dernières.

### Exemple 2 : discrimination E. coli / entérobactéries

### 2.1. Milieux et micro-organismes

13 souches bactériennes dont 3 *E. coli* et 10 entérobactéries n'appartenant pas à l'espèce *E*. *coli* ont été testées sur des milieux contenant une combinaison de substrats de phosphatase et de beta-glucuronidase. La lecture des boites est ensuite effectuée à 24h et 48h. La composition des milieux est présentée dans le Tableau 3 ci-dessous :

**Tableau 3**

| *Base commune à tous les milieux :* | |
|---|---|
| **Composés** | **Conc. en g/l** |
| **Peptone de gélatine porcine** | **5.5** |
| **Extrait de levure** | **8** |
| **agar européen** | **12.5** |
| **Chlorure de sodium** | **5** |
| **Glucuronate** | **0.2** |
| **Désoxycholate de sodium** | **0.7** |
| **Citrate de fer ammoniacal** | **0.3** |
| **Cristal violet** | **0,001** |
| **Niaproof 4** | **0.55 ml/l** |

Ensuite, l'on ajoute (en additif) les combinaisons de substrats suivantes :
- Milieu 1 : 100 mg/L de X-β-GUR et 300 mg/L de Rose-phosphate (Rose-P),
- Milieu 2 : 100 mg/L de X-β-GUR et 200 mg/L de Magenta-phosphate (Magenta-P),
- Milieu 3 : 200 mg/L de Rose-β-GUR et 100 mg/L de X-phosphate (X-P),
- Milieu 4 : 200 mg/L de Rose-β-GUR et 200 mg/L de Blue-phosphate (Blue-P),
- Milieu 5 : 300 mg/L de magenta-β-GUR et 100 mg/L de X-phosphate (X-P),
- Milieu 6 : 300 mg/L de magenta-β-GUR et 200 mg/L de Blue-phosphate (Blue-P).

### 2.2. Essais

Les milieux sont maintenus à 55°C afin d'effectuer un ensemencement en masse.
Pour cela, des suspensions bactériennes de 0.5 McF sont préparées à partir de pré-cultures de 24h sur gélose trypcase soja, incubées à 30 ou 37°C selon les souches. Ces suspensions sont ensuite diluées au 1/250000^{e} et 300µl sont déposés au fond des boites de 90 mm de diamètre puis recouverts de 18 ml de milieu.

Des lectures sont effectuées après 24h à 37°C.

### 2.3. Résultats

Les résultats obtenus sur les différents milieux, après 24h d'incubation à 37°C, sont présentés dans le Tableau 4 ci-après :

**Tableau 4**

| | | Milieu 1 X-β-GUR + Rose-P | Milieu 2 X-β-GUR + Magenta-P | Milieu 3 Rose-β-GUR + X-P | Milieu 4 Rose-β-GUR + Blue-P | Milieu 5 magenta-β-GUR + X-P | Milieu 6 magenta-β-GUR + Blue-P |
|---|---|---|---|---|---|---|---|
| *E. coli* | Coloration | Violette | Verte | Rose | Rose | Violette | Violette |
| | % coloré | 100 | 100 | 100 | 100 | 100 | 100 |
| Entérobactéries (non *E. coli*) | Coloration | Rose | Violette | Verte | Bleue | Verte | Bleue |
| | % coloré | 100 | 100 | 90 | 90 | 90 | 90 |

### 2.4. Interprétation

Tous les milieux permettent une bonne distinction entre les *E. coli* et les entérobactéries. Sur les milieux 3 à 6, seule une souche de *Shigella spp* donne une coloration proche de celle attendue pour les *E. coli,* cette souche possédant une activité beta-glucuronidase. D'un point de vue génomique, les souches de *Shigella* appartiennent à l'espèce *E. coli.* Qui plus est, le fait de « bloquer » un lot alimentaire contenant des shigelles en raison d'un soupçon de présence *d'E. coli* n'est pas problématique outre mesure, les shigelles ayant un pouvoir pathogène reconnu.

### 2.5. Conclusion

La combinaison de substrats de phosphatase et de β-glucuronidase permet bien la distinction des *E. coli* à β-glucuronidase positive et des entérobactéries sur un même milieu. En effet, les colonies d'entérobactéries exhibent une couleur correspondant à la partie marqueur du substrat de phosphatase utilisé (rose pour le Rose-P, violet pour le Magenta-P, etc.). En revanche, les colonies d'*E. coli* exhibent une tierce coloration, résultant du mélange entre la couleur produite par la partie marqueur du substrat de β-glucuronidase et celle produite par la partie marqueur du substrat de phosphatase.

### Exemple 3 : Evaluation d'un couple de substrats chromogènes sur un large panel de souches bactériennes et comparaison avec le milieu REBECCA® d'AES (mentionné dans le préambule de présente demande)

### 3.1. Milieu et micro-organismes

86 souches (cf. annexes 1 et 2), dont 5 *E. coli,* 48 entérobactéries, 23 Gram négatif non-entérobactéries, 8 Gram positif et 2 levures, ont été testées sur un milieu prototype (comprenant du Rose-β-GUR et du Blue-phosphate) et sur le milieu REBECCA® d'AES (milieu pour la détection et/ou le dénombrement des *E. coli* β-glucuronidase positives et des entérobactéries).
La lecture des boites est ensuite effectuée à 24h.
Le milieu REBECCA® est un milieu en flacon, il doit être régénéré et additionné d'un supplément avant utilisation.

Le milieu prototype est présenté dans le Tableau 5 ci-dessous :

**Tableau 5**

| **Composés** | **Conc. en g/l** |
|---|---|
| **Peptone de gélatine porcine** | **5.5** |
| **Extrait de levure** | **7** |
| **agar européen** | **12.5** |
| **Chlorure de sodium** | **5** |
| **Citrate de fer ammoniacal** | **0.3** |
| **Glucuronate** | **0.2** |
| **Glucose** | **0.1** |
| **TRIS** | **0.4** |
| **Rose-β-GUR** | **0.175** |
| **Blue-Phosphate** | **0.175** |
| **Désoxycholate de sodium** | **0.6** |
| **Cristal violet** | **0,001** |
| **Niaproof 4** | **0.3 ml/l** |

### 3.2. Essais

Les milieux sont maintenus à 55°C afin d'effectuer un ensemencement en masse.

Pour cela, l'on prépare des suspensions bactériennes de 0.5 McF à partir de pré-cultures de 24h sur TSA incubées à 30 ou 37°C selon les souches. Ces suspensions sont ensuite diluées au 1/250000^{e} et 300µl sont déposés au fond des boites de 90 mm de diamètre puis recouverts de 18 ml de milieu.

Des lectures sont effectuées après 24h à 37°C.

### 3.3. Résultats

Les résultats sont présentés dans le Tableau 6 ci-dessous :

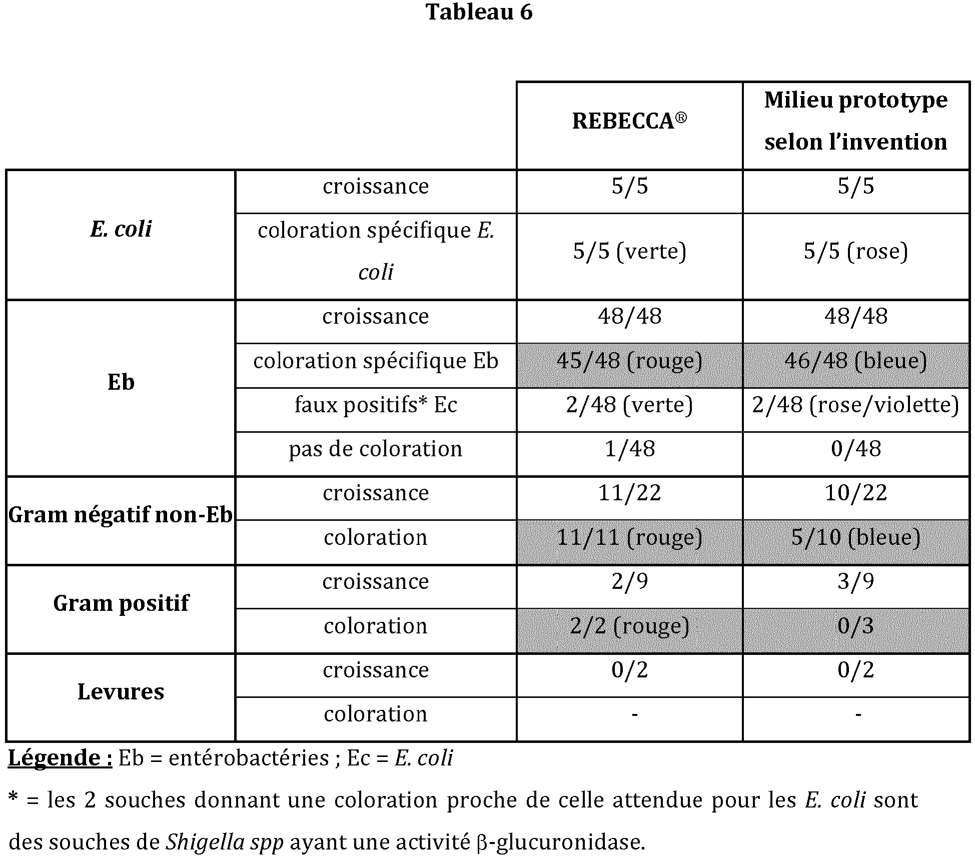

### 3.4. Interprétation

Sur les 2 milieux, l'on observe une bonne fertilité pour les souches d' *E. coli* et des entérobactéries et une sélectivité équivalente (environ 2/3 des souches non-*E*. *coli* et non-entérobactéries sont inhibées dans les deux cas).

En revanche, l'on observe une meilleure spécificité du milieu prototype selon l'invention pour les entérobactéries par rapport au milieu REBECCA®. En effet, sur les 13 souches autres que *E. coli* ou entérobactéries ayant poussé sur le milieu REBECCA®, 100% présentent une coloration rouge alors que seulement 38% sont colorées en bleu sur le milieu prototype.

### 3.5. Conclusion

L'utilisation combinée des substrats Blue-phosphate et Rose- β-glucuronide permet d'obtenir une très bonne spécificité pour la détection et la discrimination des *E. coli* β-glucuronidase positives et des entérobactéries. Cette spécificité est, contre toute attente, supérieure à celle observée sur le milieu REBECCA® d'AES (seul milieu commercialisé à ce jour pour une application de ce type).

La présente invention peut être mise en œuvre en milieu liquide, notamment pour l'analyse de l'eau. Aux fins d'une analyse de cette nature, l'on peut employer les substrats enzymatiques suivants :
- au moins un substrat nitrophenyl-phosphate (substrat de phosphatase) et au moins un substrat 4-méthyl-umbelliféryl-beta-D-glucuronide (substrat de beta glucuronidase) ;
- au moins un substrat p-aminophenyl-phosphate (substrat de phosphatase) et au moins un substrat alpha-naphtol-beta-glucuronide (substrat de beta glucuronidase) ;
- au moins un substrat X-phosphate (substrat de phosphatase) et au moins un substrat p-nitrophenyl-beta-glucuronide (substrat de beta glucuronidase).

### ANNEXE 1 - Liste des souches E.coli et Entérobactéries utilisées dans l'Exemple 3

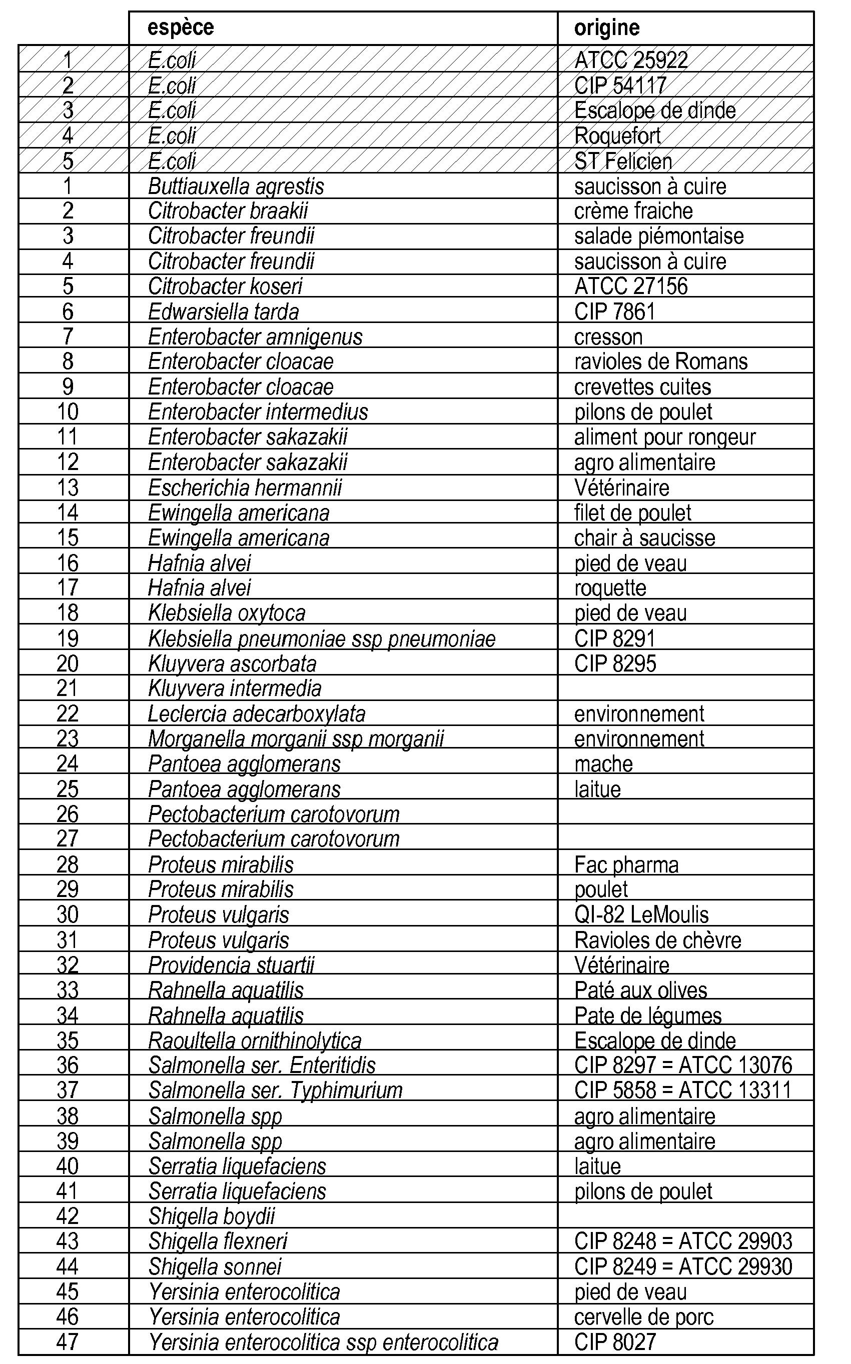

### ANNEXE 2 - Liste des souches Gram négatif, Gram positif et levures utilisées dans l'Exemple 3

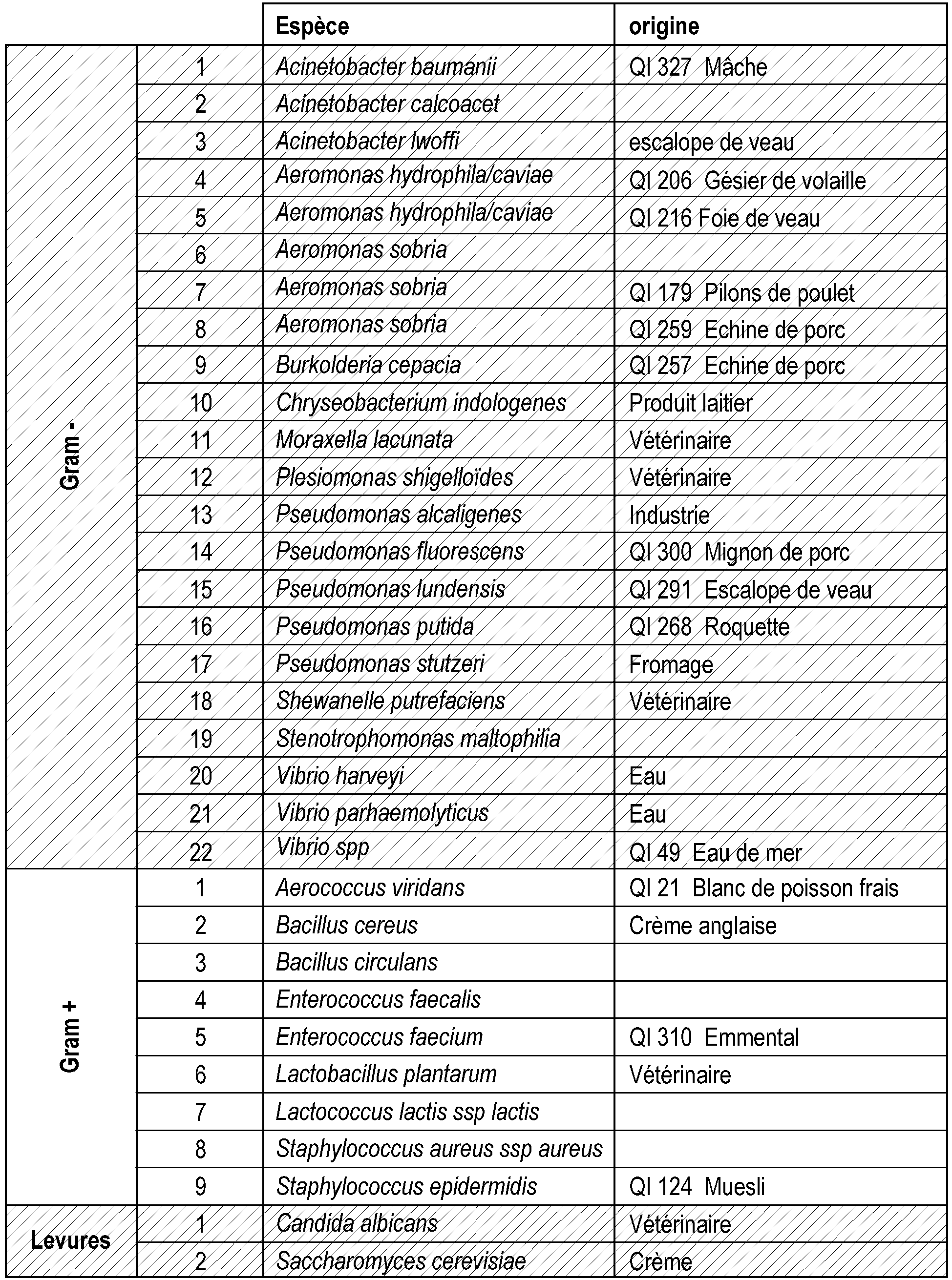

## Revendications

1. Utilisation d'au moins un substrat de phosphatase chromogène et/ou fluorogène pour la détection et/ou le dénombrement d'entérobactéries dans un échantillon susceptible de les contenir, tel qu'un échantillon alimentaire et, de manière simultanée ou séquentielle, d'au moins un substrat de β-glucuronidase chromogène et/ou fluorogène pour identifier, parmi les entérobactéries détectées via ledit substrat de phosphatase, les entérobactéries β-glucuronidase positives, par exemple les *E.coli* β-glucuronidase positives.

2. Utilisation selon la revendication 1, dans laquelle ledit au moins un substrat de phosphatase chromogène et/ou fluorogène est un substrat synthétique comprenant deux parties, une première partie spécifique de l'activité phosphatase et une seconde partie marqueur chromogène et/ou fluorogène.

3. Utilisation selon la revendication 1 ou 2, dans laquelle ledit substrat de phosphatase est sélectionné parmi :
- les substrats à base d'indoxyl ou d'un de ses dérivés, tels que le 5-bromo-4-chloro-3-indoxyl-phosphate, le 5-bromo-6-chloro-3-indoxyl-phosphate, le 6-chloro-3-indoxyl-phosphate, le 5-iodo-3-indoxyl-phosphate, le 6-bromo-3-indoxyl-phosphate, le 5,6-dibromo-3-indoxyl-phosphate, le 5-bromo-3-indoxyl-phosphate, le Aldol® 458 phosphate, le Aldol® 470 phosphate, le Aldol® 484 phosphate, le Aldol®495 phosphate, le Aldol®515 phosphate, le Aldol® n phosphate, et
- le 3-hydroxyflavone-phosphate.

4. Utilisation selon l'une des revendications 1 à 3, dans laquelle ledit substrat de phosphatase est un substrat à base d'indoxyl ou d'un de ses dérivés, ledit substrat de phosphatase étant utilisé en combinaison avec au moins un agent favorisant la polymérisation oxydative du dérivé indoxyl, tel qu'un complexe métallique de type citrate de fer ammoniacal, de préférence ledit substrat de phosphatase étant choisi parmi le 5-bromo-4-chloro-3-indoxyl-phosphate, le 5-bromo-6-chloro-3-indoxyl-phosphate, le 6-chloro-3-indoxyl-phosphate, le 5-iodo-3-indoxyl-phosphate, le 6-bromo-3-indoxyl-phosphate, le 5,6-dibromo-3-indoxyl-phosphate, le 5-bromo-3-indoxyl-phosphate.

5. Utilisation selon l'une des revendications 1 à 4, dans laquelle ledit substrat de phosphatase est contenu dans une composition comprenant au moins un, de préférence deux, et avantageusement trois des constituants suivants :
- un milieu de culture bactérien adapté aux bactéries à détecter,
- un système sélectif anti-Gram positif,
- un agent antifongique.

6. Utilisation selon l'une des revendications 1 à 5, dans laquelle ledit substrat de β-glucuronidase chromogène et/ou fluorogène est un substrat synthétique comprenant deux parties, une première partie spécifique de l'activité β-glucuronidase, et une seconde partie marqueur chromogène et/ou fluorogène.

7. Utilisation selon l'une des revendications 1 à 6, dans laquelle ledit substrat de β-glucuronidase est sélectionné parmi :
- le 4 méthylumbelliféryl-β-glucuronide,
- les substrats à base d'indoxyl ou d'un de ses dérivés, tels que le 5-bromo-4-chloro-3-indoxyl-β-glucuronide, le 5-bromo-6-chloro-3-indoxyl-β-glucuronide, le 6-chloro-3-indoxyl-β-glucuronide, de préférence le 6-chloro-3-indoxyl-β-glucuronide,
- l'alizarine-β**-**glucuronide et le cyclohexenoesculetine-β-glucuronide ou leurs sels, de préférence ledit au moins un substrat de β-glucuronidase étant au moins un substrat à base d'indoxyl ou d'un de ses dérivés, avantageusement le 6-chloro-3-indoxyl-β-glucuronide.

8. Utilisation selon l'une des revendications 1 à 7, dans laquelle ledit substrat de phosphatase et/ou ledit substrat de β-glucuronidase est/sont un/des substrat(s) à base d'indoxyl ou un de ses dérivés utilisé(s) en combinaison avec au moins un agent favorisant la polymérisation oxydative du dérivé indoxyl tel qu'un complexe métallique de type citrate de fer ammoniacal.

9. Utilisation selon l'une des revendications 1 à 8, dans laquelle ledit substrat de phosphatase et ledit substrat de β-glucuronidase sont contenus dans une composition comprenant au moins un, de préférence deux, et avantageusement trois des constituants suivants :
- un milieu de culture bactérien adapté aux bactéries à détecter,
- un système sélectif anti-Gram positif,
- un agent antifongique.

10. Utilisation d'une composition comprenant au moins un substrat de phosphatase chromogène et/ou fluorogène et au moins un substrat de β-glucuronidase chromogène et/ou fluorogène pour détecter la présence des entérobactéries dans un échantillon via leur activité phosphatase et identifier, parmi les entérobactéries ainsi détectées, les entérobactéries β-glucuronidase positives via leur activité β-glucuronidase, par exemple les *E.coli* β-glucuronidase positives.

11. Utilisation selon la revendication 10, dans laquelle ledit au moins un substrat de phosphatase chromogène et/ou fluorogène est tel que défini dans la revendication 2 ou 3 et/ou ledit substrat de β-glucuronidase est tel que défini dans la revendication 6 ou 7.

12. Utilisation selon la revendication 10 ou 11, dans laquelle ledit substrat de phosphatase et/ou ledit substrat de β-glucuronidase est/sont un/des substrat(s) à base d'indoxyl ou d'un de ses dérivés, ladite composition comprenant au moins un agent favorisant la polymérisation oxydative du dérivé indoxyl dudit substrat de phosphatase et/ou dudit substrat de β-glucuronidase, tel qu'un complexe métallique de type citrate de fer ammoniacal.

13. Utilisation selon l'une des revendications 10 à 12, ladite composition comprenant au moins un, de préférence deux, et avantageusement trois des constituants suivants :
- un milieu de culture bactérien adapté aux bactéries à détecter,
- un système sélectif anti-Gram positif,
- un agent antifongique.

14. Procédé de détection et/ou de dénombrement d'entérobactéries dans un échantillon susceptible de les contenir, ledit procédé comprenant les étapes suivantes :
a) Prélever ledit échantillon,
b) Mettre en contact l'échantillon prélevé à l'étape a) avec une composition telle que définie dans l'une des revendications 10 à 13,
c) Eventuellement incuber l'ensemble à une température et durant un laps de temps adaptés pour permettre la croissance desdites entérobactéries,
d) Détecter et/ou dénombrer :
- les entérobactéries, de préférence leur(s) colonie(s), présentant une première coloration et/ou fluorescence produite(s) par l'activité phosphatase desdites entérobactéries sur ledit substrat de phosphatase et, de manière simultanée ou séquentielle, de préférence de manière simultanée,
- les entérobactéries β-glucuronidase positives, par exemple les *E.coli* β-glucuronidase positives, de préférence leur(s) colonie(s), présentant une coloration et/ou fluorescence composite produite(s) par le mélange de ladite première coloration et/ou fluorescence et d'une deuxième coloration et/ou fluorescence produite(s) par l'activité β-glucuronidase desdites entérobactéries β-glucuronidase positives.

## Patentansprüche

1. Eine Verwendung von mindestens einem chromogenen und/oder fluorogenen Phosphatasesubstrat für den Nachweis und/oder die Zählung von Enterobakterien in einer Probe, die sie enthalten kann, wie beispielsweise eine Lebensmittelprobe, und auf gleichzeitige oder aufeinanderfolgende Weise von mindestens einem chromogenen und/oder fluorogenen β-Glucuronidasesubstrat zum Identifizieren, aus den über das Phosphatasesubstrat nachgewiesenen Enterobakterien, der β-Glucuronidase-positiven Enterobakterien, beispielsweise der β-Glucuronidase-positiven *E. coli.*

2. Verwendung gemäß Anspruch 1, wobei das mindestens eine chromogene und/oder fluorogene Phosphatasesubstrat ein synthetisches Substrat ist, das zwei Teile, einen ersten für die Phosphataseaktivität spezifischen Teil und einen zweiten chromogenen und/oder fluorogenen Marker-Teil, beinhaltet.

3. Verwendung gemäß Anspruch 1 oder 2, wobei das Phosphatasesubstrat ausgewählt ist aus Folgendem:
- Substraten auf Basis von Indoxyl oder einem seiner Derivate, wie 5-Brom-4-chlor-3-indoxylphosphat, 5-Brom-6-chlor-3-indoxylphosphat, 6-Chlor-3-indoxylphosphat, 5-lod-3-indoxylphosphat, 6-Brom-3-indoxylphosphat, 5,6-Dibrom-3-indoxylphosphat, 5-Brom-3-indoxylphosphat, Aldol®-458-Phosphat, Aldol®-470-Phosphat, Aldol®-484-Phosphat, Aldol®-495-Phosphat, Aldol®-515-Phosphat, Aldol®-n-Phosphat und
- 3-Hydroxyflavonphosphat.

4. Verwendung gemäß einem der Ansprüche 1 bis 3, wobei das Phosphatasesubstrat ein Substrat auf Basis von Indoxyl oder einem seiner Derivate ist, wobei das Phosphatasesubstrat in Kombination mit mindestens einem die oxidative Polymerisation des Indoxylderivats fördernden Mittel verwendet wird, wie beispielsweise einem Metallkomplex vom Eisenammoniumcitrat-Typ, wobei das Phosphatasesubstrat vorzugsweise aus 5-Brom-4-chlor-3-indoxylphosphat, 5-Brom-6-chlor-3-indoxylphosphat, 6-Chlor-3-indoxylphosphat, 5-Iod-3-indoxylphosphat, 6-Brom-3-indoxylphosphat, 5,6-Dibrom-3-indoxylphosphat, 5-Brom-3-indoxylphosphat gewählt ist.

5. Verwendung gemäß einem der Ansprüche 1 bis 4, wobei das Phosphatasesubstrat in einer Zusammensetzung enthalten ist, die mindestens einen, vorzugsweise zwei und vorteilhafterweise drei der folgenden Bestandteile beinhaltet:
- ein an die nachzuweisenden Bakterien angepasstes Bakterienkulturmedium,
- ein anti-grampositiv-selektives System,
- ein Antimykotikum.

6. Verwendung gemäß einem der Ansprüche 1 bis 5, wobei das chromogene und/oder fluorogene β-Glucuronidasesubstrat ein synthetisches Substrat ist, beinhaltend zwei Teile, einen ersten für β-Glucuronidaseaktivität spezifischen Teil und einen zweiten chromogenen und/oder fluorogenen Marker-Teil.

7. Verwendung gemäß einem der Ansprüche 1 bis 6, wobei das β-Glucuronidasesubstrat aus Folgendem ausgewählt ist:
- 4-Methylumbelliferyl-β-glucuronid,
- Substraten auf Basis von Indoxyl oder einem seiner Derivate, wie 5-Brom-4-chlor-3-indoxyl-β-glucuronid, 5-Brom-6-chlor-3-indoxyl-β-glucuronid, 6-Chlor-3-indoxyl-ß-glucuronid, vorzugsweise 6-Chlor-3-indoxyl-β-glucuronid,
- Alizarin-ß-glucuronid und Cyclohexenesculetin-β-glucuronid oder deren Salze, vorzugsweise wobei das mindestens eine β-Glucuronidasesubstrat mindestens ein Substrat auf Basis von Indoxyl oder einem seiner Derivate, vorteilhafterweise 6-Chlor-3-indoxyl-β-glucuronid, ist.

8. Verwendung gemäß einem der Ansprüche 1 bis 7, wobei das Phosphatasesubstrat und/oder das β-Glucuronidasesubstrat ein Substrat/Substrate auf Basis von Indoxyl oder einem seiner Derivate ist/sind, das/die in Kombination mit mindestens einem die oxidative Polymerisation des Indoxylderivats fördernden Mittel verwendet wird/werden, wie beispielsweise einem Metallkomplex vom Eisenammoniumcitrat-Typ.

9. Verwendung gemäß einem der Ansprüche 1 bis 8, wobei das Phosphatasesubstrat und das β-Glucuronidasesubstrat in einer Zusammensetzung enthalten sind, die mindestens einen, vorzugsweise zwei und vorteilhafterweise drei der folgenden Bestandteile beinhaltet:
- ein an die nachzuweisenden Bakterien angepasstes Bakterienkulturmedium,
- ein anti-grampositiv-selektives System,
- ein Antimykotikum.

10. Eine Verwendung einer Zusammensetzung, beinhaltend mindestens ein chromogenes und/oder fluorogenes Phosphatasesubstrat und mindestens ein chromogenes und/oder fluorogenes β-Glucuronidasesubstrat zum Nachweisen des Vorhandenseins von Enterobakterien in einer Probe über deren Phosphataseaktivität und zum Identifizieren, unter den so nachgewiesenen Enterobakterien, β-Glucuronidase-positiver Enterobakterien über deren β-Glucuronidaseaktivität, zum Beispiel β-Glucuronidase-positive *E. coli.*

11. Verwendung gemäß Anspruch 10, wobei das mindestens eine chromogene und/oder fluorogene Phosphatasesubstrat wie in Anspruch 2 oder 3 definiert ist und/oder das β-Glucuronidasesubstrat wie in Anspruch 6 oder 7 definiert ist.

12. Verwendung gemäß Anspruch 10 oder 11, wobei das Phosphatasesubstrat und/oder das β-Glucuronidasesubstrat ein Substrat/Substrate auf Basis von Indoxyl oder einem seiner Derivate ist/sind, wobei die Zusammensetzung mindestens ein die oxidative Polymerisation des Indoxylderivats des Phosphatasesubstrats und/oder des β-Glucuronidasesubstrats förderndes Mittel, wie ein Metallkomplex vom Eisenammoniumcitrat-Typ, beinhaltet.

13. Verwendung gemäß einem der Ansprüche 10 bis 12, wobei die Zusammensetzung mindestens einen, vorzugsweise zwei und vorteilhafterweise drei der folgenden Bestandteile beinhaltet:
- ein an die nachzuweisenden Bakterien angepasstes Bakterienkulturmedium,
- ein anti-grampositiv-selektives System,
- ein Antimykotikum.

14. Ein Verfahren zum Nachweisen und/oder Zählen von Enterobakterien in einer Probe, die diese enthalten kann, wobei das Verfahren die folgenden Schritte beinhaltet:
a) Entnehmen der Probe,
b) In-Kontakt-Bringen der in Schritt a) entnommenen Probe mit einer Zusammensetzung wie in einem der Ansprüche 10 bis 13 definiert,
c) optional Inkubieren des Ganzen bei einer Temperatur und während eines Zeitraums, die angepasst sind, um das Wachstum der Enterobakterien zu ermöglichen,
d) Nachweisen und/oder Zählen von Folgendem:
- Enterobakterien, vorzugsweise deren Kolonie(n), die eine erste Färbung und/oder Fluoreszenz aufweist/aufweisen, die durch die Phosphataseaktivität der Enterobakterien auf dem Phosphatasesubstrat und auf gleichzeitige oder aufeinanderfolgende Weise, vorzugsweise gleichzeitige Weise, erzeugt wurde/n,
- β-Glucuronidase-positiven Enterobakterien, zum Beispiel β-Glucuronidase-positive *E. coli,* vorzugsweise deren Kolonie(n), die eine Färbung und/oder Fluoreszenz aufweist/aufweisen, die durch das Mischen der ersten Färbung und/oder Fluoreszenz und einer zweiten Färbung und/oder Fluoreszenz erzeugt wird/werden, die durch die β-Glucuronidaseaktivität der β-Glucuronidase-positiven Enterobakterien erzeugt wird/werden.

## Claims

1. Use of at least one chromogenic and/or fluorogenic phosphatase substrate for the detection and/or enumeration of enterobacteria in a sample likely to contain them, such as a food sample and, simultaneously or sequentially, of at least one chromogenic and/or fluorogenic β-glucuronidase substrate to identify, among the enterobacteria detected via said phosphatase substrate, the β**-**glucuronidase-positive enterobacteria, for example β**-**glucuronidase-positive *E.coli.*

2. Use according to claim 1, wherein said at least one chromogenic and/or fluorogenic phosphatase substrate is a synthetic substrate comprising two parts, a first part specific to phosphatase activity and a second chromogenic and/or fluorogenic marker part.

3. Use according to claim 1 or 2, wherein said phosphatase substrate is selected from:
- substrates based on indoxyl or on one of its derivatives, such as 5-bromo-4-chloro-3-indoxyl-phosphate, 5-bromo-6-chloro-3-indoxyl-phosphate, 6-chloro-3-indoxyl-phosphate, 5-iodo-3-indoxyl-phosphate, 6-bromo-3-indoxyl-phosphate, 5,6-dibromo-3-indoxyl-phosphate, 5-bromo-3-indoxyl-phosphate, Aldol® 458 phosphate, AIdol® 470 phosphate, Aldol® 484 phosphate, Aldol® 495 phosphate, Aldol®515 phosphate, AIdol® n phosphate, and
- 3-hydroxyflavone-phosphate.

4. Use according to one of claims 1 to 3, wherein said phosphatase substrate is a substrate based on indoxyl or on one of its derivatives, said phosphatase substrate being used in combination with at least one agent promoting oxidative polymerisation of the indoxyl derivative, such as a metal complex of the ammonium ferric citrate type, preferably said phosphatase substrate being chosen from 5-bromo-4-chloro-3-indoxyl-phosphate, 5-bromo-6-chloro-3-indoxyl-phosphate, 6-chloro-3-indoxyl-phosphate, 5-iodo-3-indoxyl-phosphate, 6-bromo-3-indoxyl-phosphate, 5,6-dibromo-3-indoxyl-phosphate, 5-bromo-3-indoxyl-phosphate.

5. Use according to one of claims 1 to 4, wherein said phosphatase substrate is contained within a composition comprising at least one, preferably two, and advantageously three of the following constituents:
- a bacterial culture medium suited to the bacteria to be detected,
- an anti-Gram-positive selective system,
- an antifungal agent.

6. Use according to one of claims 1 to 5, wherein said chromogenic and/or fluorogenic β-glucuronidase substrate is a synthetic substrate comprising two parts, a first part specific to β-glucuronidase activity, and a second chromogenic and/or fluorogenic marker part.

7. Use according to one of claims 1 to 6, wherein said β-glucuronidase substrate is selected from:
- 4 methylumbelliferyl-β-glucuronide,
- substrates based on indoxyl or on one of its derivatives, such as 5-bromo-4-chloro-3-indoxyl-β-glucuronide, 5-bromo-6-chloro-3-indoxyl-β-glucuronide, 6-chloro-3-indoxyl-β-glucuronide, preferably 6-chloro-3-indoxyl-β-glucuronide,
- alizarin-β-glucuronide and cyclohexenoesculetin-β-glucuronide or salts thereof, preferably said at least one β-glucuronidase substrate being at least one substrate based on indoxyl or on one of its derivatives, advantageously 6-chloro-3-indoxyl-β-glucuronide.

8. Use according to one of claims 1 to 7, wherein said phosphatase substrate and/or said β-glucuronidase substrate is/are one or more substrate(s) based on indoxyl or on one of its derivatives used in combination with at least one agent promoting oxidative polymerisation of the indoxyl derivative, such as a metal complex of the ammonium ferric citrate type.

9. Use according to one of claims 1 to 8, wherein said phosphatase substrate and said β-glucuronidase substrate are contained in a composition comprising at least one, preferably two, and advantageously three of the following constituents:
- a bacterial culture medium suited to the bacteria to be detected,
- an anti-Gram-positive selective system,
- an antifungal agent.

10. Use of a composition comprising at least one chromogenic and/or fluorogenic phosphatase substrate and at least one chromogenic and/or fluorogenic β-glucuronidase substrate to detect the presence of enterobacteria in a sample via their phosphatase activity and identify, among the enterobacteria thus detected, the β-glucuronidase-positive enterobacteria via their β-glucuronidase activity, for example β-glucuronidase-positive *E.coli.*

11. Use according to claim 10, wherein said at least one chromogenic and/or fluorogenic phosphatase substrate is as defined in claim 2 or 3 and/or said β-glucuronidase substrate is as defined in claim 6 or 7.

12. Use according to claim 10 or 11, wherein said phosphatase substrate and/or said β-glucuronidase substrate is/are one or more substrate(s) based on indoxyl or on one of its derivatives, said composition comprising at least one agent promoting oxidative polymerisation of the indoxyl derivative of said phosphatase substrate and/or of said β-glucuronidase substrate, such as a metal complex of the ammonium ferric citrate type.

13. Use according to one of claims 10 to 12, said composition comprising at least one, preferably two, and advantageously three of the following constituents:
- a bacterial culture medium suited to the bacteria to be detected,
- an anti-Gram-positive selective system,
- an antifungal agent.

14. Method of detection and/or enumeration of enterobacteria in a sample likely to contain them, said method comprising the following steps:
a) Take said sample,
b) Bring the sample taken in step a) into contact with a composition as defined in one of claims 10 to 13,
c) Possibly incubate the assembly at a temperature and for a period of time suitable to allow the growth of said enterobacteria,
d) Detect and/or enumerate:
- the enterobacteria, preferably their colony (colonies), exhibiting a first coloration and/or fluorescence produced by the phosphatase activity of said enterobacteria on said phosphatase substrate, and simultaneously or sequentially, preferably simultaneously,
- the β**-**glucuronidase-positive enterobacteria, for example β-glucuronidase-positive *E.coli,* preferably their colony (colonies), exhibiting a composite coloration and/or fluorescence produced by the mixture of said first coloration and/or fluorescence and a second coloration and/or fluorescence produced by the β-glucuronidase activity of said β**-**glucuronidase-positive enterobacteria.
